# FASCICULE DE BREVET EUROPEEN

(11) **EP 1 861 695 B1**
(45) Date de publication et mention de la délivrance du brevet: **02.09.2009**
(21) Numéro de dépôt: 06726135.4
(22) Date de dépôt: 23.03.2006
(51) Int. Cl.: G01N 21/64, G01N 33/566, C07J 9/00

(54) **SONDES PYRENIQUES MIMANT LE CHOLESTEROL**
CHOLESTERIN IMITIERENDE PYRENSONDEN
CHOLESTEROL-MIMETIC PYRENE PROBES

(30) Priorité: 23.03.2005 FR 0502884
(43) Date de publication de la demande: 05.12.2007
(73) Titulaire: CENTRE NATIONAL DE LA RECHERCHE SCIENTIFIQUE, 75794 Paris Cedex 16 (FR)
(72) Inventeur: LE ROUX, Christophe, F-31500 Toulouse (FR); LOPEZ, André, F-31400 Toulouse (FR)
(74) Mandataire: Mouget-Goniot, Claire
(86) Numéro de dépôt international: PCT/FR2006/000641
(87) Numéro de publication internationale: WO 2006/100388

(56) Documents cités:
- LAGANE ET AL: "Lateral distribution of cholesterol in membranes probed by means of a pyrene-labelled cholesterol: effects of acyl chain unsaturation" BIOPHYSICAL CHEMISTRY, NORTH-HOLLAND, AMSTERDAM, NL, vol. 95, 2002, pages 7-22, XP002360396 ISSN: 0301-4622 cité dans la demande
- LI ET AL: "Effect of cholesteryl ester on the distribution of fluorescent cholesterol analogues in triacylglycerol-rich emulsions" BBA - LIPIDS AND LIPID METABOLISM, ELSEVIER SCIENCE BV. AMSTERDAM, NL, vol. 1166, 1993, pages 145-153, XP002360397 ISSN: 0005-2760
- KAO ET AL: "N-(2-Naphthyl)-23,24-dinor-5-cholen-22-am in-3.beta.-ol, a fluorescent cholesterol analogue" BIOCHEMISTRY, AMERICAN CHEMICAL SOCIETY. EASTON, PA, US, vol. 17, no. 13, 1978, pages 2689-2696, XP002360398 ISSN: 0006-2960

## Description

L'invention concerne des sondes membranaires mimant les propriétés physiques du cholestérol, et l'utilisation de ces sondes au sein de membranes plasmiques naturelles afin de caractériser grâce à une réponse photophysique l'organisation collective des lipides de la membrane.

L'étude de l'organisation fonctionnelle des membranes biologiques est aujourd'hui une problématique d'un grand intérêt [1]. Les approches expérimentales proposant des avancées dans ce domaine sont basées sur des extractions sélectives à froid de sous-ensembles membranaires résistants à l'action de certains tensio-actifs [2] appelés « rafts ». Les résultats de ces expériences sont fort critiquables car les échantillons extraits pourraient correspondre à des regroupements réalisés lors de l'extraction de microdomaines membranaires insolubles appartenant peut-être à des membranes cellulaires différentes [3]. Ainsi, toute corrélation entre fonction et composition initiale des membranes est impossible et aucune organisation structurale des molécules ne peut être générée sans ambiguïté par ces approches biochimiques. La seule certitude qu'elles apportent est que les membranes forment des assemblages multi-moléculaires composites, ayant des propriétés physiques moyennes différentes, qui induisent des spécificités d'activation pour les systèmes protéiques membranaires qu'elles renferment. Ainsi, les membranes ne sont pas uniquement des barrières entre l'extérieur et l'intérieur des cellules, car grâce au polymorphisme des lipides et au rôle particulier des stérols, un regain d'intérêt s'y focalise avec l'introduction entre autre de la notion de « rafts » lipidiques [4] sous-ensembles de la membrane plasmique qui participent à la modulation des fonctions de signalisation réalisées par les protéines surfaciques cellulaires.

Sur membranes modèles, les propriétés des phases enrichies en cholestérol ou celles des phases riches en phospholipides constitués d'acides gras insaturés sont connues et documentées [5-8]. En quelques mots, les zones « rafts » seraient des zones lipidiques membranaires plus épaisses que les zones fluides de type liquide désordonnée notées 1d ou 1α. Ces zones « rafts » correspondraient à des zones de phases lipidiques « liquide ordonnée » notées 1o, contraintes rotationnellement et conformationnellement, composées de forts pourcentages en cholestérol et lipides à chaînes grasses saturées, et ayant une mobilité translationnelle réduite d'un facteur 2 à 3 par rapport aux zones lipidiques 1α. Il est à noter que ces états d'organisation lipidiques de type 1α étaient jusqu'à ces dernières années reconnus comme les seuls pouvant exister parmi les différents types d'organisations lipidiques. Cette description statique des membranes doit être complétée par le fait que ces différentes zones membranaires « raft » et non « raft » ont leurs constituants en échange permanent. Cette dynamique translationnelle de la majorité des constituants membranaires est une des caractéristiques générales des membranes. L'ensemble de ces données fait qu'à ce jour, il n'existe pas de méthode permettant d'établir pour une membrane plasmique naturelle donnée, le taux de phase 1o et 1α. Or, la connaissance de ces valeurs ou d'une grandeur qui leur serait proportionnelle renseignerait sur l'importance de ces « rafts » au sein d'une membrane : suivant le type cellulaire, ces « rafts » pourraient représenter de 20 à 60% de la surface totale d'une membrane plasmique ! [9]. De plus, il est admis que certaines fonctions se réalisent spécifiquement dans des zones « raft » et d'autres réciproquement dans des zones non « raft » [1, 4], alors que les protéines membranaires participant à ces fonctions sont retrouvées dans les deux types de zones [10]. Cette problématique sur l'organisation fonctionnelle des machineries plurimoléculaires assurant les fonctions essentielles à la vie de toute cellule a fait naître la notion,de transducisome, c'est à dire des sous ensembles membranaires qui seraient des « plateaux techniques optimisés » où se réaliserait une fonction donnée. Leur bonne connaissance est un des enjeux majeurs de la biologie cellulaire moderne.

Enfin, pour compléter cette description biologique du transducisome, il faut ajouter les propriétés conformationnelles des protéines transmembranaires lors de la réalisation de leur fonction. Elles introduisent un lien logique avec les propriétés de phase des lipides énumérées précédemment. En effet, des changements conformationnels de ces protéines transmembranaires sont observés lors de la réalisation de leur fonction: Les travaux de Salamon et coll. sur la rhodopsine [11] ou sur le récepteur δ aux opioïdes [12], mettent en évidence qu'une protéines transmembranaire peut changer de 10% son épaisseur apparente lors de la liaison d'un agoniste et ne rien modifier à ce paramètre de structure lors de la liaison d'un antagoniste. Ce changement d'épaisseur pourrait être corrélé soit:
(i) à des variations par tri moléculaire de la distribution latérale des constituants membranaires [13, 14] et regrouper ainsi des constituants de la membrane d'une épaisseur adéquate avec celle de la protéine dans une conformation fonctionnelle donnée et ceci afin de minimiser un mésappariement hydrophobe entre protéine et lipides,
(ii) à l'induction d'une migration de cette protéine vers des zones plus épaisses (« rafts ») après liaison de l'agoniste et de la protéine G (dans le cas des exemples de Salamon et al. [11, 12] ), car ce changement conformationnel lors de la signalisation cellulaire par ces RCPG (Récepteurs Couplés aux Protéines G) implique une augmentation de l'épaisseur de ces derniers [11, 12].

En relation avec cette problématique les inventeurs ont conduit dans le passé des études sur membranes naturelles sur le récepteur aux opioïdes hMOR et ont montré une modulation de sa fonctionnalité par les lipides (cholestérol) de la membrane plasmique quand il est exprimé dans des cellules eucaryotes (Saccharomyces Cerevisiae SC [15] ou cellules d'Ovaires d'Hamster Chinois (CHO) [16] ). Ainsi l'induction par la composition membranaire d'un état « quasi auto constitutif, » pour ce RCPG, capable de lier la protéine G sans la liaison de l'agoniste a été établi.

Les inventeurs ont également utilisé une molécule commerciale dérivée du cholestérol: le 1-pyrèneméthyl-3β -hydroxy-22, 23-bisnor-5-cholate (Py-CO2-Chol) et ont montré[17] que cette sonde incorporée par construction dans des liposomes est capable de rendre compte de la distribution latérale du cholestérol [16, 17]. De plus, il a également été montré que cette sonde est un révélateur sensible de l'état de phase moyen des membranes modèles dans lesquelles elle est insérée. Les extrêmes seraient une membrane où les lipides sont dans un état de phase de type 1α (membrane caractérisée par une faible épaisseur) d'une part ou une membrane dont l'état de phase des lipides est de type 1o (membrane à forte épaisseur) d'autre part. Ainsi, par des mesures en fluorescence stationnaire du rapport des bandes 1 et 3 du spectre de fluorescence du monomère du fluorophore pyrène, il est possible de remonter à une caractérisation de l'état de phase moyen [16]. Cette propriété est caractéristique du pyrène non substitué [18]. Elle est perdue avec les phospholipides à pyrène [19] et retrouvée avec les composés de la famille Py-Chol.

Cependant, cette sonde commerciale, le Py-CO2-Chol, pose un problème de possibilité de marquage des membranes plasmiques naturelles. Sa faible solubilité dans les lipides membranaires comparée à celle du cholestérol (7% au lieu de 68% dans le DMPC) en est la cause. La liaison de jonction ester entre les groupements pyrène et cholestérol (de nature polaire) coupant les parties hydrophobes de ce composé, pourrait être responsable de ce fait [17]. L'invention apporte une solution à l'impossibilité d'effectuer ce type de mesures sur cellules vivantes avec des molécules fluorescentes commerciales mimant le cholestérol.

Par ailleurs, cette approche en fluorescence stationnaire de la compartimentation des membranes naturelles a été complétée par des mesures des paramètres caractéristiques de la dynamique des constituants membranaires: les expériences de SPT (Single Particle Tracking) [21-23] ou de FRAPrv (Fluorescence Recovery after photobleaching at variable radius) [24, 25] ont prouvé l'existence de structurations dynamiques latérales de taille submicrométrique, et ceci in situ sur cellules. Ces deux approches dynamiques sont complémentaires (observations individuelles en SPT ou analyse d'un comportement collectif en FRAPrv). Ainsi :
i) Par SPT les déplacements de récepteurs hMOR individuels, à la surface de cellules NRK; ont mis en évidence un mode de diffusion translationnelle de hMOR combinant un mode de diffusion confinée superposé à une marche aléatoire du confinement. [21-22].
ii) Une analyse statistique approfondie des résultats a permis de développer un modèle théorique explicatif des confinements observés et des interactions qui pourraient les générer [21-22]. Il introduit un nouveau type de confinement sans barrière, uniquement imposé par les interactions entre entités membranaires.
iii) La notion de transducisome et de sa spécificité d'évolution ainsi que son importance surfacique sur un type de cellule ont été démontrées par FRAPrv sur de cellules HEK et pour la fonction des RCPG à la neurokine [25].

L'invention a principalement pour but de fournir des sondes moléculaires pouvant s'insérer efficacement dans les membranes biologiques des cellules *in vivo,* notamment par le biais de méthyl-β-cydodextrines, et qui par leur réponse sont capables de rendre compte, des variations de distributions latérales moyennes des constituants membranaires.

A ce titre, les inventeurs sont parvenus à synthétiser de nouveaux composés mixtes à partir de cholestérol et de pyrène, ayant à la jonction entre le cholestérol et le pyrène un groupement cétone ou méthylénique, avec un bras de liaison de longueur variable.

De manière surprenante, les inventeurs ont mis en évidence que non seulement les composés pyrène-cétone-cholestérol et pyrène-méthylénique-cholestérol sont d'excellents révélateurs de l'état de phase moyen des lipides, de par leurs propriétés de fluorescence, mais aussi que la propension de ces composés à une insertion dans les membranes naturelles est remarquable.

L'invention a principalement pour objet les composés ci-après dénommés pyrène-cholestérol, de formule générale (I) : 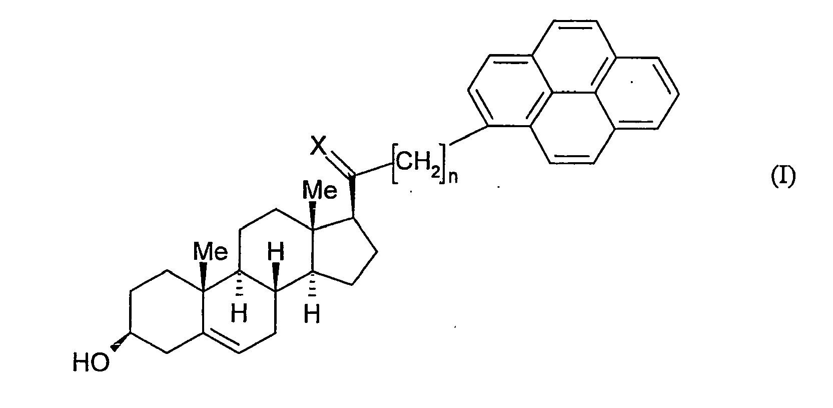 dans laquelle X représente un groupe CH₂ ou un atome d'oxygène et n est un nombre entier de 2 à 10, notamment de 2 à 6.

L'invention concerne plus particulièrement un composé de formule générale (I) dénommé pyrène-cétone-cholestérol, de formule (4) suivante :

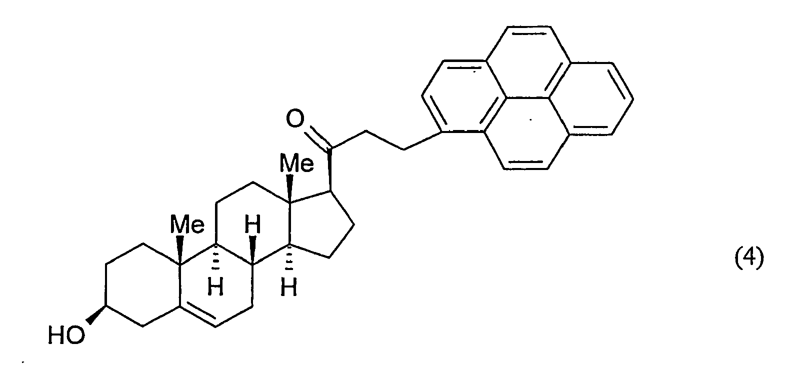

L'invention a plus particulièrement pour objet encore un composé de l'invention de formule générale (I) dénommé pyrène-méthylénique-cholestérol, de formule (7) suivante :

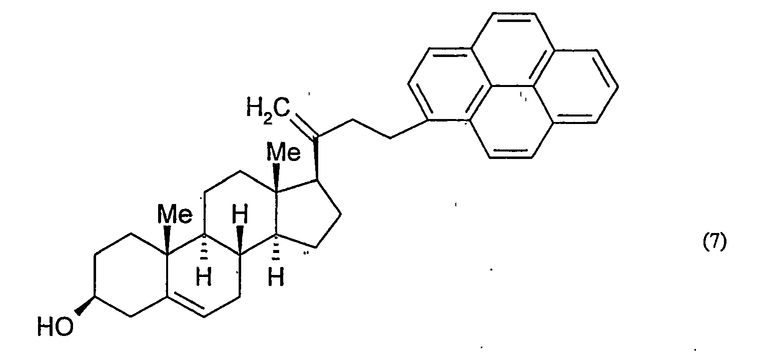

L'invention concerne également l'utilisation d'un composé pyrène-cholestérol de formule (I) défini précédemment, ou d'une combinaison de ces composés pyrène-cholestérol, pour l'étude de l'organisation fonctionnelle des membranes biologiques, et de ses changements sous l'effet de stimuli extérieurs, tels que la température, la pression, l'ajout d' agent extrinsèque modifiant la concentration d'un lipide contenu initialement dans lesdites membranes biologiques, ou l'ajout d'un produit déterminé.

Par « organisation fonctionnelle des membranes biologiques » pour une fonction biologique donnée, on entend les paramètres lipidiques caractéristiques de la structure de la membrane comme l'état de phase des lipides (liquide ordonné 1o ou liquide désordonné 1d) et/ou de distribution latérale des constituants de la membrane qui participent à l'optimisation de la fonction biologique donnée.

Des exemples connus d'agents extrinsèques susceptibles de moduler la concentration en un lipide donné des membranes biologiques, sont des composés de la famille des cyclodextrines qui déplètent spécifiquement les membranes en certains - lipides ou d'autres agents lipophiles extrinsèques qui par insertion modulent la fluidité membranaire (rigidification ou fluidification).[26] L'invention a plus particulièrement pour objet l'utilisation susmentionnée d'un composé pyrène-cholestérol de formule (I) dans le cadre de la mise en oeuvre d'une méthode de détection, et, le cas échéant, de mesure de la polarité ou la perméabilité des membranes biologiques, de la concentration d'un ou de plusieurs constituants de ces membranes, et ainsi l'invention permet de rendre compte des changements de l'organisation spatiale et collective des constituants de ces membranes biologiques induits par ces facteurs extrinsèques.

Des exemples de constituants des membranes dont la présence peut être détectée ou la concentration mesurée selon l'invention sont le cholestérol ou des récepteurs membranaires couplés aux protéines G (RCPG) lors du déclanchement de leur signalisation par un ligand. Par «organisation spatiale et collective des constituants des membranes biologiques » on entend le type de phase lipidique moyen représentatif des états de phase de la membrane avant et après activation de RCPG par l'agent extrinsèque.

L'invention concerne plus particulièrement l'utilisation susmentionnée d'un composé pyrène-cholestérol de formule (I) ci-dessus, dans le cadre de la mise en oeuvre d'une méthode de détection, et, le cas échéant, de mesure photophysique des effets de produits déterminés sur les membranes biologiques, ladite méthode étant appliqués :
- au criblage de produits bioactifs susceptibles d'être des agonistes ou des antagonistes de récepteurs présents dans des membranes cellulaires ou à la surface de ces membranes, lesdits produits bioactifs étant susceptibles d'être utilisés pour la préparation de médicaments destinés au traitement de pathologies dans lesquelles interviennent lesdits récepteurs ;
- au diagnostic *in vitro* de pathologies à partir de cellules de patients à l'aide de produits déterminés utilisés en tant que marqueurs de ces pathologies, notamment de cellules susceptibles d'être sensibles à un produit déterminé utilisé en tant que marqueur, et dont l'effet sur ces cellules est caractéristique d'une pathologies,
- au contrôle de l'orientation de l'indication thérapeutique d'un médicament comprenant un produit bioactif tel que défini ci-dessus, par détermination in vitro de l'effet agoniste ou antagoniste de ce médicament sur les cellules du patient susceptibles d'être traité par ce médicament ; avantageusement, cette méthode de contrôle permet d'orienter le médecin vers un traitement personnalisé le plus adapté possible à un patient déterminé, avec un produit bioactif ainsi sélectionné.

Des, exemples de produits bioactifs susceptibles d'être criblés grâce à l'invention sont des ligands agonistes ou antagoniste de RCPG. Ces derniers étant préalablement surexprimés dans les membranes plasmiques des cellules biologiques.

Des exemples de pathologies contre lesquelles peuvent être préparés des médicaments à partir des produits bioactifs criblés selon l'invention sont toutes les pathologies dont l'agent responsable est un récepteur membranaire de la famille des RCPG (60% des cibles pharmacologiques actuelles).

Selon un mode de réalisation préférée des utilisations susmentionnées d'un composé pyrène-cholestérol de formule (I) selon l'invention, les membranes biologiques sont choisies parmi le groupe constitué de vésicules, de liposomes unilamellaires, de liposomes multilamellaires, de bicouches ou de multicouches lipidiques supportées, de membranes cellulaires extraites ou de membranes cellulaires de cellules vivantes.

Par « multicouches lipidiques supportées » on entend des membranes modèles correspondant à la superposition de une, deux ou plusieurs bicouches lipidiques et qui sont des modèles membranaires performants pour les études et mesures photophysiques.

Avantageusement, les membranes cellulaires susmentionnées sont celles des cellules eucaryotes suivantes : CHO (cellule d'ovaire d'hamster chinois), HEK (cellule endothéliale humaine de rein), NRK (cellule endothéliale de rein de rat) AEB (cellule endothéliale aortique bovine), AEH (cellule endothéliale aortique humaine), SHY5Y (neuroblastome), 3T3, cultures primaires de neurones, de fibrobastes, ou issues d'autres organes ou tissus.

L'invention a également pour objet un procédé pour l'étude de l'organisation fonctionnelle des membranes biologiques, et la mise en oeuvre d'une méthode de détection, et, le cas échéant, de mesure des effets de stimuli extérieurs, tels que la température, la pression, la modulation par un agent extrinsèque de la concentration en un lipide contenu initialement dans lesdites membranes biologiques, ou l'ajout d'un produit déterminé influençant l'organisation desdites membranes biologiques, **caractérisé en ce qu**'il comprend :
- une étape d'incorporation d'un composé de formule (I) défini ci-dessus, ou d'une association de plusieurs de ces composés, dans lesdites membranes biologiques,
- le cas échéant l'application de stimuli extérieurs tels que définis ci-dessus, sur lesdites membranes, étape sautée pour les mesures de référence,
- l'application sur lesdites membranes biologiques d'un rayonnement de longueur d'onde λₑₓ caractéristique de l'absorption du pyrène, λₑₓ étant compris entre 330 nm et 350 nm,
- la mesure de l'intensité du rayonnement de fluorescence émis par lesdites membranes biologiques à la longueur d'onde d'émission de 375 à 385 nm, caractéristique de la bande d'émission 1 du pyrène, cette intensité étant fonction de la polarité de la membrane,
- la mesure de l'intensité d'au moins un des deux rayonnements de fluorescence émis par lesdites membranes biologique entre 390 nm et 425 nm, caractéristiques des bandes d'émission 3 et 4 du pyrène, cette intensité étant fonction de la quantité de pyrène, et donc de composés selon l'une des revendications 1 à 3, dans lesdites membranes,
- la détermination du rapport entre l'intensité de la bande 1 et celle de la bande 3 et/ou 4, à savoir des rapports I₁/I₃ et/ou I₁/I₄,
- la corrélation entre le rapport mesuré lors de l'étape précédente, et le niveau de polarité desdites membranes qui est fonction de leur épaisseur, donnant ainsi une indication sur le type d'organisation fonctionnelle moyenne de ces membranes et de son évolution sous l'effet d'un stimuli extérieur.

Avantageusement, le procédé susmentionné est appliqué au criblage de produits bioactifs susceptibles d'être des agonistes ou des antagonistes de récepteurs présents dans des membranes cellulaires ou partiellement insérés dans ces membranes, lesdits produits bioactifs étant susceptibles d'être utilisés pour la préparation de médicaments destinés au traitement de pathologies dans lesquelles interviennent lesdits récepteurs, ledit procédé comprenant :
- une étape d'incorporation d'un composé de formule (I) défini ci-dessus, ou d'une association de plusieurs de ces composés, dans lesdites membranes biologiques,
- la mise en présence desdites membranes avec un produit bioactif, ou une banque de produits bioactifs, dans des conditions permettant la liaison de ces produits avec les récepteurs membranaires,
- le cas échéant, une étape de rinçage desdites membranes pour éliminer tout produit bioactif qui ne se serait pas susceptible de se lier avec un récepteur membranaire,
- l'application sur lesdites membranes biologiques d'un rayonnement de longueur d'onde λₑₓ caractéristique de l'absorption du pyrène, λₑₓ étant compris entre 330 nm et 350 nm,
- la mesure de l'intensité des bandes 1, 3, et/ou 4, et la détermination des rapports I₁/I₃ et/ou I₁/I₄,
- la comparaison des rapports I₁/I₃ et/ou I₁/I₄, obtenus à l'étape précédente avec les rapports I₁/I₃ et/ou I₁/I₄, mesurés en l'absence de produits bioactifs, ce qui permet de déterminer la nature agoniste ou antagoniste ou antagoniste inverse du ou des produits bioactifs testés, un produit agoniste étant caractérisé par un rapport I₁/I₃ et/ou I₁/I₄ respectivement supérieur ou inférieur au rapport I'₁/I'₃ et/ou I'₁/I'₄ suivant le type desdits récepteurs, un produit antagoniste étant caractérisé par un rapport I₁/I₃ et/ou I₁/I₄ équivalent respectivement au rapport I'₁/I'₃ et/ou I'₁/I'₄, un produit antagoniste inverse étant caractérisé par un rapport I₁/I₃ et/ou I₁/I₄ respectivement inférieur ou supérieur au rapport I'₁/I'₃ et/ou I'₁/I'₄ soit un effet inverse à celui d'un produit agoniste.

L'invention concerne plus particulièrement un procédé tel que défini ci-dessus **caractérisé en ce qu**'il est effectué *in vitro* sur des cellules eucaryotes, et comprend une étape d'incorporation d'un composé de formule (I) de l'invention, ou d'une association de plusieurs de ces composés, dans les membranes de ces cellules.

Avantageusement, dans les procédés susmentionnés de l'invention, l'étape d'incorporation dans lesdites membranes d'un composé de l'invention, ou de l'association de plusieurs de ces composés, s'effectue au moyen d'un transporteur destiné à échanger le cholestérol, un phospholipide, naturellement présents dans lesdites membranes, avec ledit composé pyrène-cholestérol ou ladite combinaison.

De manière préférée, les procédés définis ci-dessus sont **caractérisés en ce que** le transporteur est une méthyl-β-cyclodextrine, ou une α-, β-, γ-cyclodextrine.

L'invention a plus particulièrement pour objet un procédé tel que défini ci-dessus, **caractérisé en ce que** la proportion de composé de formule (I) de l'invention, ou de l'association de plusieurs de ces composés, incorporée dans lesdites membranes représente environ 0,1 à 2 % de la concentration surfacique des lipides présents dans la membrane biologique.

L'invention a également pour objet une composition **caractérisée en ce qu**'elle comprend un composé de formule (I) de l'invention, ou une association de plusieurs de ces composés, en mélange avec un transporteur destiné à échanger le cholestérol ou un phospholipide, naturellement présents dans lesdites membranes avec ledit composé pyrène-cholestérol ou ladite combinaison.

De manière préférée, cette composition est **caractérisée en ce que** le transporteur est une méthyl-β-cyclodextrine, ou une α-, β-, γ-cyclodextrine.

L'invention a également pour objet l'utilisation de la composition définie ci-dessus dans l'étape d'incorporation d'un composé de l'invention, ou d'une association de plusieurs de ces composés, dans lesdites membranes biologiques, lors de la mise en oeuvre d'un procédé tel que défini ci-dessus.

L'invention a également pour objet un procédé de synthèse des composés définis ci-dessus, **caractérisé en ce qu**'il comprend les étapes suivantes :
a) la réaction de la prégnènolone de formule (a) et du dérivé du pyrène de formule (b) dans laquelle n est un nombre entier de 2 à 10, notamment de 2 à 6, en présence de EtOK, EtOH, dans CH₂Cl₂ à une température comprise entre 5°C et 20°C et pendant une durée comprise entre 10 et 72 heures, ce qui conduit à l'obtention du composé de formule (1) suivant : 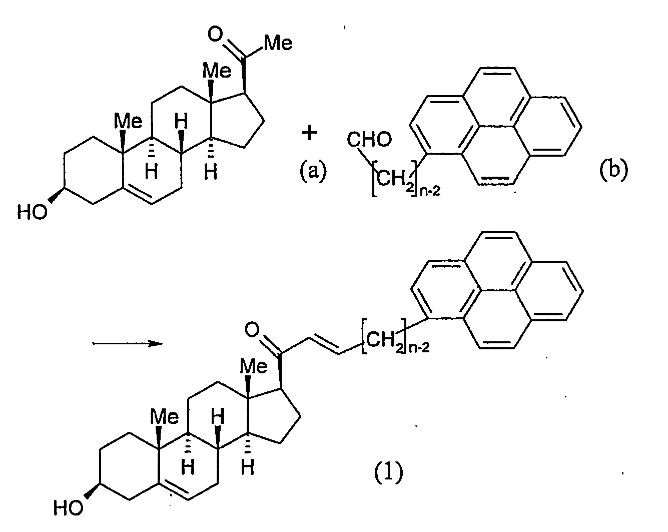
b) la réaction du composé de formule (1) obtenu à l'étape précédente, en présence de 1,5 équivalents de Ac₂O, de 10 mol % de DMAP, de 1 équivalent de Et₃N, dans CH₂Cl₂ pendant 3 heures, ce qui conduit à l'obtention du composé de formule (2) suivant : 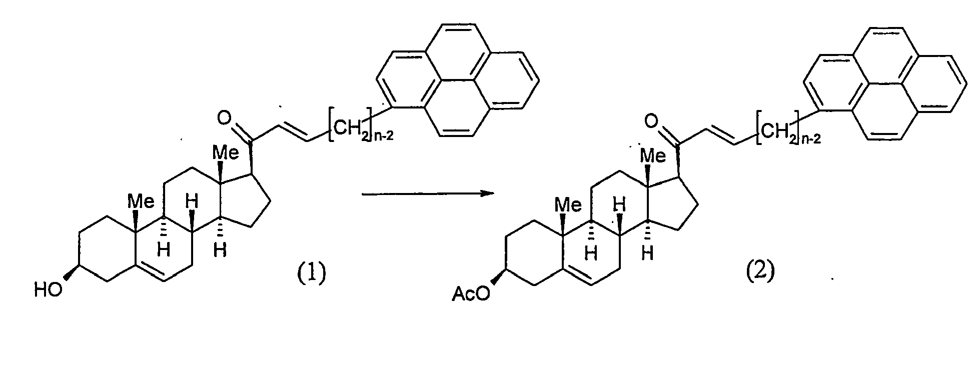
c) la réaction du composé de formule (2) obtenu à l'étape précédente en présence de 2 équivalents de TiCl₄, de 1,5 équivalents de Et₃SiH, dans CH₂Cl₂ pendant 45 minutes à température ambiante, ce qui conduit à l'obtention du composé de formule (3) suivant : 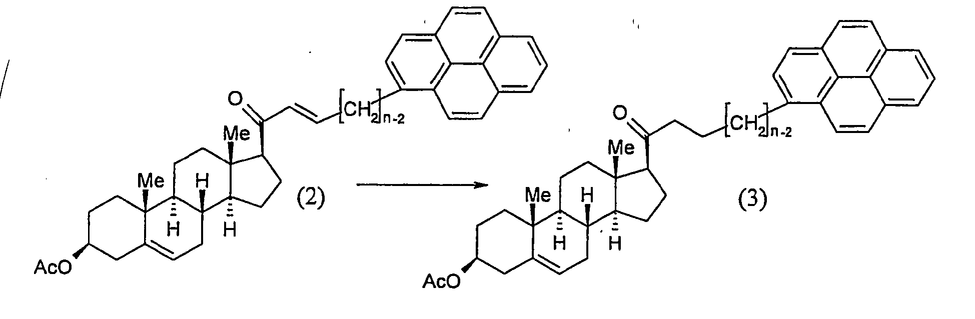
d) la réaction du composé de formule (3) obtenu à l'étape précédente en présence de MeONa, CH₂Cl₂ pendant 4 heures à température ambiante, conduit an composé de formule (4) suivant : 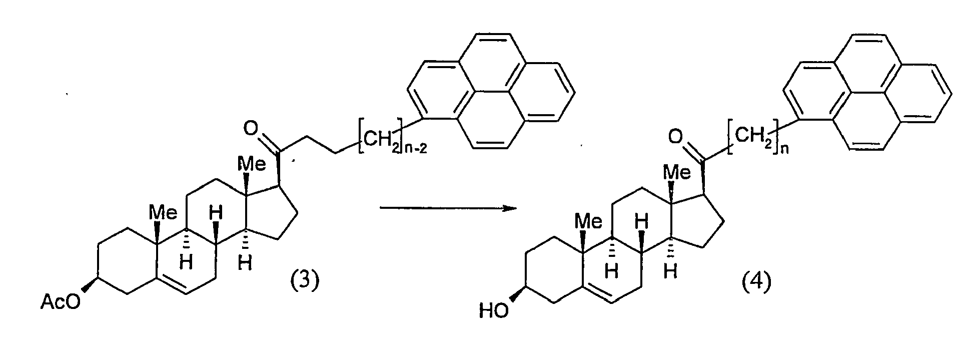
e) le cas échéant, la réaction du composé de formule (4) obtenu à l'étape précédente en présence de TBDMSCI, d'imidazole, dans CH₂Cl₂ pendant 12 heures à température ambiante, ce qui conduit à l'obtention du composé de formule (5) suivant: 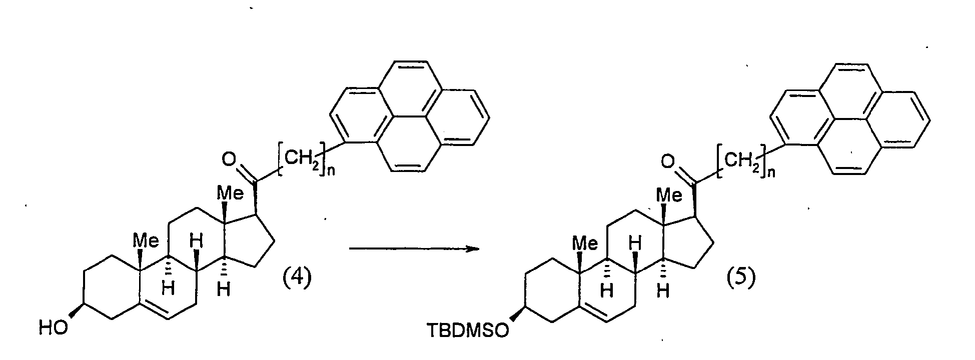
f) la réaction du composé de formule (5) obtenu à l'étape précédente en présence de 2 équivalents de CH₂=PPh₃, dans un mélange THF/DMSO, pendant 12 heures à 75°C, ce qui conduit à l'obtention du composé de formule (6) suivant : 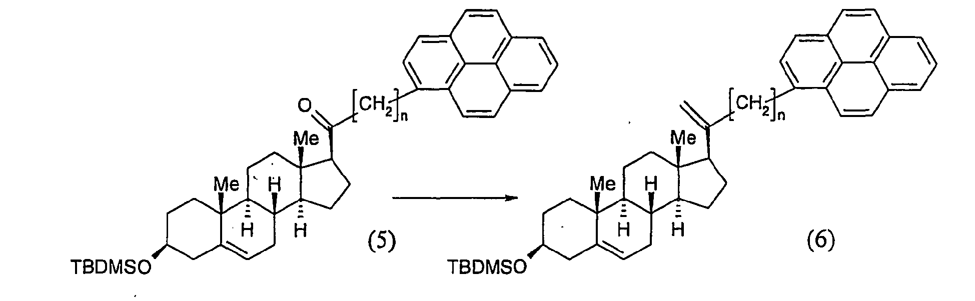
g) la réaction du composé de formule (6) obtenu à l'étape précédente en présence de 5 équivalents de TBAF, dans le THF, pendant 12 heures à 65°C, au composé de formule (7) suivant :

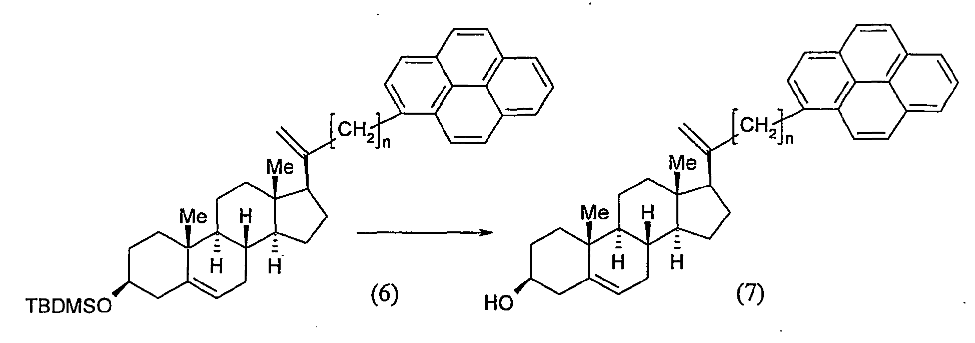

### Description des figures

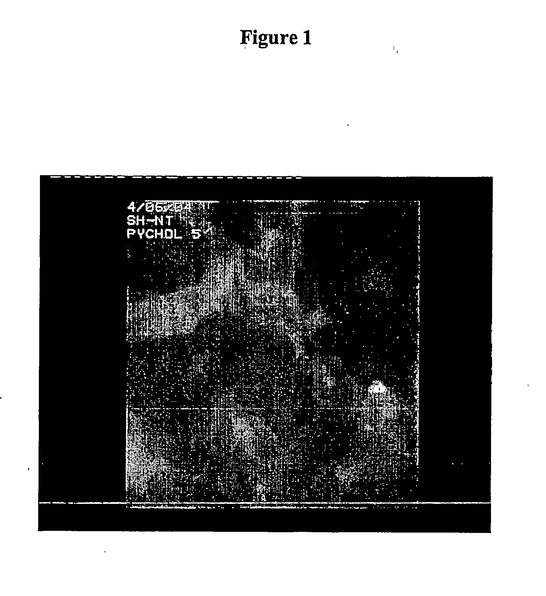

La figure 1 représente le marquage des membranes de cellules SH-SY5Y in vivo au moyen de méthyl-β-cyclodextrines chargées en sonde pyrène-cétone-cholestérol. L'insertion peut se faire à plus de 40 % et est fonction du temps de marquage.

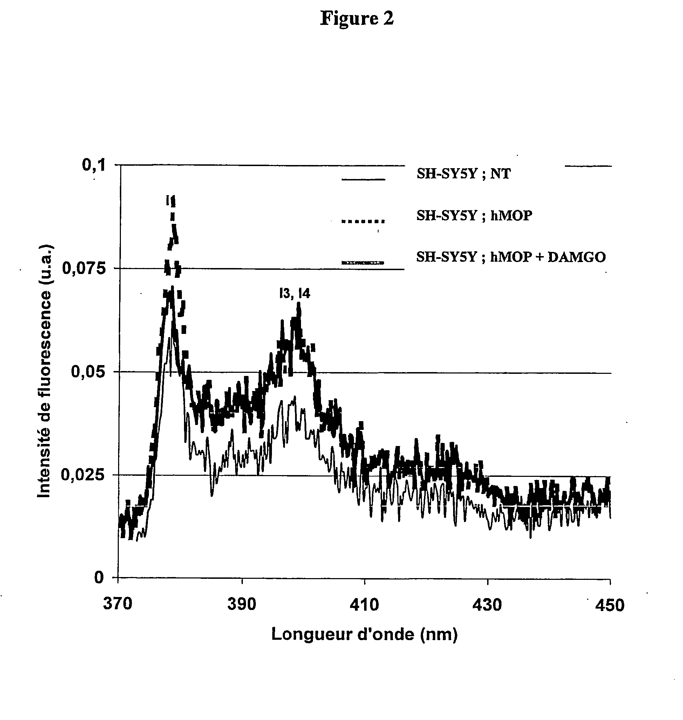

La figure 2 représente les spectres de fluorescence réalisés sur cellules SH-SY5Y immobilisées sur support solide (lamelle de silice) après incorporation dans la membrane plasmique, à l'aide de méthyl-β-cyclodextrine, de la sonde pyrène-cétone-cholestérol de formule (4). En trait fin continu noir, les cellules non transfectées par le RCPG µ aux opïoides (hMOP) ; en trait épais pointillé noir, les cellules transfectées par ce dernier ; en trait épais continu gris, l'échantillon précédent sur lequel l'agoniste DAMGO a été ajouté en conditions saturantes.

### PARTIE EXPERIMENTALE

Tous les produits chimiques commerciaux sont utilisés tels que reçus. Toutes les réactions sont effectuées sous argon en utilisant la technique standard de Schlenk. Les données de RMN proton et carbone 13 sont obtenues avec un dispositif Bruker Avance 300. Les spectrogrammes de masse sont obtenus avec un appareil NERMAG R10-10 en conditions d'ionisation chimique avec de l'ammoniac ou du méthane. Les spectres infrarouges sont obtenus sur un spectrophotomètre FTIR Perkin Elmer de série 1600 sous forme de pastilles de KBr et sont indiqués en cm⁻¹. Les points de fusion ne sont pas corrigés.

### Synthèse de la (21E)-3β-hydroxy-prégna-5,21-diène-22-(1-pyrényl)-20-one (composé 1)

A une solution de 1-pyrènecarboxaldéhyde (3,2 g, 14 mmol) et de prégnènolone (4 g, 12,64 mmol) dans 30 mL de CH₂Cl₂ sec, on ajoute 15 mL d'éthanol sec puis 25 mL d'une solution d'éthanolate de potassium à 24 % en masse dans de l'éthanol, et la solution est agitée pendant 72 h à +5°C. On ajoute 200 mL d'eau et on extrait les produits organiques avec du CH₂Cl₂ (3 x 100 mL). Les extraits organiques combinés sont rincés avec de la saumure, séchés sur du sulfate de sodium, filtrés et évaporés. La purification par chromatographie flash sur silice G60 (0,040-0,063 mm), l'élution avec de l'acétate d'éthyle dans du CH₂Cl₂ (2/98) puis avec de l'acétate d'éthyle dans du CH₂Cl₂ (15/85) donnent 6,12g (91%) de composé **1** sous forme d'un solide jaune brillant ; mp: 153°C; TLC (CH₂Cl₂/AcOEt 15:85) *R_{f}* 0.5; IR (KBr, cm⁻¹) 3300m, 2932s, 1676m, 1590s, 1436w, 1370w, 1352w, 1315w, 1234w, 1192w, 1102m, 1052m, 973w, 848s, 709w; ¹H RMN (CDCl₃) δ 0.59 (s, 3H), 0.87 (s, 3H), 0.70-2.38 (m, 22H), 2.73 (t, *J* = 8.7 Hz, 1H), 3.42 (sept, *J* = 4.8 Hz, 1H) 3.55 (t, *J* = 9 Hz, 2H), 5.24 (d, *J* = 4.8 Hz, 1H), 6.87 (d, *J* = 15.6 Hz, 1H), 7.87-8.17 (m, 8H), 8.36 (d, *J* = 9.3 Hz, 1H), 8.58 (d, *J* = 15.6 Hz, 1H), ¹³C RMN (CDCl₃) δ 200.2, 140.8, 138.0, 132.7, 131.3, 130.7, 130.2, 128.7, 128.6, 128.5, 127.3, 126.3, 126.0, 125.8, 125.0, 124.9, 124.6, 124.0, 122.5, 121.4, 71.7, 62.5, 57.1, 53.5, 50.0, 45.1, 42.3, 39.2, 37.3, 36.5, 32.0, 31.8, 31.6, 24.7, 22.8, 21.1, 19.4, 13.5; MS (CI, NH₃) m/z 529 (100, [M + H]⁺).

### Synthèse de la (21E)-3β-acétoxy-prégna-5,21-diène-22-(1-pyrényl)-20-one (composé 2).

A une solution du composé **1** (1,3 g, 2,46 mmol) dans 20 mL de CH₂Cl₂, on ajoute successivement de l'anhydride acétique (1 mL, 5,28 mmol), de la 4-(diméthylamino)pyridine (30 mg, 246 µmol) et de la triéthylamine (343 µL, 2,46 mmol). La solution obtenue est agitée pendant 3h à température ambiante, puis on ajoute de l'eau (150 mL) et on extrait les produits organiques avec du CH₂Cl₂ (3 x 80 mL). Les extraits combinés sont séchés sur du sulfate de sodium, concentrés sous pression réduite et donnent après chromatographie sur colonne (CH₂Cl₂/AcOEt 98/2) 1,16 g (83%) de composé **2** sous forme de solide jaune ; mp 99°C; TLC (AcOEt/CH₂Cl₂ 2:98) *R_{f}* 0.9; IR (KBr, cm⁻¹) 2938s, 1728s, 1676w, 1590s, 1371m, 1242s, 1102m, 1126m, 845s; ¹H RMN (CDCl₃) δ 0.58 (s, 3H), 0.87 (s, 3H), 1.95 (s, 3H), 0.70-2.36 (m, 21H), 2.71 (t, *J* = 9 Hz, 1H), 4.52 (m, 1H), 5.24 (d, *J* = 4.8 Hz, 1H), 6.85 (d, *J* = 15.6 Hz, 1H), 7.87-8.17 (m, 8H), 8.36 (d, *J* = 9.6 Hz, 1H), 8.56 (d, *J* = 15.9 Hz, 1H); ¹³C RMN (CDCl₃) δ 200.1, 170.6, 139.6, 137.9, 132.7, 131.3, 130.7, 130.2, 128.7, 128.6, 128.5, 127.3, 126.3, 126.0, 125.8, 125.0, 124.9, 124.6, 124.0, 122.5, 122.4, 73.9, 62.4, 57.0, 49.9, 45.0, 39.1, 38.1, 37.0, 36.6, 31.9, 31.8, 27.8, 24.7, 22.8, 21.5, 21.1, 19.3, 13.5 (seules 39 des 40 résonances attendues sont observées); MS (CI, NH₃) m/z 571 (100, [M + H]⁺).

### Synthèse de la 3β-acétoxy-prégn-5-ène-21-(1-méthylpyrényl)-20-one (composé 3)

A une solution glacée du composé **2** (1 g, 1,75 mmol) dans 20 mL de CH₂Cl₂ on ajoute 4,2 mL d'une solution à 1M de TiCl₄ dans du CH₂Cl₂ et on agite la solution brun sombre qui en résulte pendant 10 min. On ajoute de l'Et₃SiH (419 µL, 2,63 mmol) d'un seul coup et on chauffe la solution à température ambiante et on l'agite pendant 45 min. La solution est versée dans une solution saturée de NaHCO₃ et les produits organiques sont extraits avec du CH₂Cl₂ (3 x 80 mL Les extraits combinés sont séchés sur du sulfate de sodium, concentrés sous pression réduite et donnent après chromatographie sur colonne (CH₂Cl₂/AcOEt 98:2) 601 mg (60%) du composé **3** sous forme d'un solide jaune pâle ; mp 91°C ; TLC (CH₂Cl₂ /AcOEt 98:2) *R_{f}* 0.9; IR (KBr, cm⁻¹) 2938s, 2902s, 1729s, 1701s, 1456w, 1436w, 1362m, 1246s, 1095w, 1031m, 906w, 845m, 731m; ¹H RMN (CDCl₃) δ 0.49 (s, 3H), 0.86 (s, 3H), 1.94 (s, 3H), 0.6-2.4 (m, 20H), 2.77 (t, *J =* 8.1 Hz, 2H), 3.51 (t, *J* = 7.5 Hz, 2H), 4.49 (m, 1H), 5.23 (d, *J* = 4.2 Hz, 1H), 7.76-8.14 (m, 9H); ¹³C RMN (CDCl₃) δ 210.5, 170.5, 139.6, 135.6, 131.4, 130.9, 130.0, 128.5, 127.6, 127.5, 127.3, 126.7, 125.9, 125.1, 125.0, 124.9, 124.8, 123.0, 122.3, 73.8, 63.1, 56.7, 49.7, 46.1, 44.2, 38.8, 38.1, 36.9, 36.5, 31.7, 31.6, 27.7, 27.4, 24.5, 23.0, 21.4, 20.9, 19.2, 13.4 (on observe seulement 39 des 40 résonances attendues); MS (CI, NH₃) m/z 590 (100, [M + NH₄]⁺), 573 (98, [M + H]⁺).

### Synthèse de la 3β-hydroxy-prégn-5-ène-21-(1-méthylpyrényl)-20-one (composé 4).

A une solution du composé **3** (600 mg, 1 mmol) dans 10 mL de CH₂Cl₂ on ajoute successivement 5 mL de méthanol sec et 5 mL d'une solution à 30 % en masse de méthanolate de sodium dans du méthanol et on agite la solution pendant 4 h à température ambiante. On ajoute 200 mL d'eau et les produits organiques sont extraits avec du CH₂Cl₂ (3 x 100 mL). Les extraits organiques combinés sont rincés avec de la saumure, séchés sur du sulfate de sodium, filtrés, et évaporés. La purification par chromatographie flash sur silice G60 (0.040-0.063 mm), l'élution avec du CH₂Cl₂ dans de l'AcOEt (85/15) donnent 302 mg (95%) de composé 4 sous forme d'un solide blanc cassé ; mp 199°C; TLC (CH₂Cl₂/AcOEt 85:15) *R_{f}* 0.5; IR (KBr, cm⁻¹) 3428m, 2930s, 1700s, 1458w, 1436w, 1357w, 1108w, 1054m, 845s, 755s; ¹H RMN (CDCl₃) δ 0.55 (s, 3H), 0.89 (s, 3H), 0.70-2.24 (m, 19H), 2.40 (t, *J* = 9 Hz, 1H), 2.84 (t, *J* = 6 Hz, 2H), 3.42 (m, 1H) 3.55 (t, *J* = 9 Hz, 2H), 5.24 (d, *J* = 4.8 Hz, 1H), 7.80-8.18 (m, 9H); ¹³C RMN (CDCl₃) δ 210.5, 140.7, 135.7, 131.4, 130.9, 130.0, 128.5, 127.6, 127.5, 127.3, 126.8, 125.9, 125.1, 125.0, 124.9, 124.8, 123.0, 121.4, 71.7, 63.2, 56.9, 49.9, 46.1, 44.3, 42.2, 38.9, 37.2, 36.5, 31.8, 31.7, 31.6, 27.4, 24.5, 23.0, 21.0, 19.3, 13.5 (on observe seulement 37 des 38 résonances attendues); MS (CI, NH₃) m/z 548 (100, [M + NH₄]⁺), 531 (17, [M + H]⁺).

### Synthèse de la 3β-tert-butyldiméthylsilyloxy-prégn-5-ène-21-(1-méthylpyrényl)-20-one (composé 5).

A une solution de composé **4** (200 mg, 377 µmol) et d'imidazole (51 mg, 754 mmol) dans 5 mL de CH₂Cl₂ on ajoute au moyen d'une seringue une solution de chlorure de tert-butyldiméthylsilyle (68 mg, 452 mmol) dans 2 mL of CH₂Cl₂. Après 12h d'agitation à température ambiante, on ajoute 50 mL d'eau et on extrait les produits organiques avec du dichlorométhane (3 x 50 mL). Les extraits organiques combinés sont rincés avec de la saumure, séchés sur du sulfate de sodium, filtrés, et évaporés. La purification par chromatographie flash sur silice G60 (0.040-0.063 mm), l'élution avec du dichlorométhane donnent 231 mg (95%) de composé 5 sous forme d'un solide blanc cassé ; mp 100°C; TLC (CH₂Cl₂) *R_{f}* 0.9; IR (KBr, cm⁻¹) 2929s, 2852s, 1703s, 1461m, 1381m, 1359m, 1250m, 1183w, 1091s, 843s, 773m; ¹H RMN (CDCl₃) δ 0.00 (s, 6H), 0.47 (s, 3H), 0.60-2.22 (m, 32H), 2.72 (t, *J* = 7.5 Hz, 2H), 3.39 (m, 1H) 3.49 (t, *J* = 7.8 Hz, 2H), 5.17 (d, *J* = 3.9 Hz, 1H), 7.73-8.12 (m, 9H); ¹³C RMN (CDCl₃) δ 210.4, 141.4, 135.7, 131.4, 130.9, 130.0, 128.5, 127.6, 127.5, 127.3, 126.8, 125.9, 125.1, 125.0, 124.9, 124.8, 123.1, 120.9, 72.6, 63.0, 56.7, 49.9, 46.0, 44.2, 42.8, 38.8, 37.3, 36.5, 32.1, 31.8, 31.7, 27.4, 26.2, 26.0, 25.8, 24.5, 23.0, 21.0, 19.4, 18.3, 13.4, -4.5 (on observe seulement 42 des 44 résonances attendues); MS (CI, CH₄) m/z 645 (100, [M + H]⁺).

### Synthèse du 3β-tert-butyldiméthylsilyloxy-prégn-5-ène-21-(1-méthylpyrényl)-20-méthylidène (composé 6).

On ajoute 48 mg de NaH (2 mmol) à 3 mL de DMSO sec et la suspension est agitée et chauffée à 65°C jusqu'à ce que tout l'hydrure de sodium soit dissout. La solution est refroidie à température ambiante et on ajoute de bromure de méthyltriphénylphosphonium (715 mg, 2 mmol) par portions pendant 5 min. La réaction est légèrement exothermique et on agite la solution pendant une durée supplémentaire de 45 min. On ajoute cette solution d'un seul coup à une solution de composé **5** (645 mg, 1 mmol) dans 5 mL de THF et la solution résultante est agitée et chauffée à 70°C pendant 12h. Après refroidissement, on ajoute 50 mL d'eau et on extrait les produits organiques avec du dichlorométhane (3 x 50 mL). Les extraits organiques combinés sont rincés avec de la saumure, séchés sur du sulfate de sodium, filtrés, et évaporés. La purification par chromatographie flash sur silice G60 (0.040-0.063 mm), l'élution avec du pentane dans du CH₂Cl₂ (75/25) donnent 514 mg (80%) de composé 6 sous forme d'un solide blanc cassé ; mp 63°C; TLC (pentane/CH₂Cl₂ 75:25) *R_{f}* 0.85; IR (KBr, cm^{-1 1}) 2928s, 2851s, 1636w, 1460m, 1434m, 1380w, 1249m, 1088s, 888m, 842s, 773m; ¹H RMN (CDCl₃) δ 0.00 (s, 6H), 0.54 (s, 3H), 0.83 (s, 9H), 0.91 (s, 3H), 0.75-2.30 (m, 32H), 2.47 (m, 2H), 3.40 (m, 3H), 4.85 (s, 1H), 5.01 (s, 1H), 5.24 (d, *J* = 6.0 Hz, 1H), 7.77-8.20 (m, 9H); ¹³C RMN (CDCl₃) δ 149.1, 141.6, 136.8, 133.9, 133.6, 131.5, 131.0, 129.8, 128.7, 128.6, 128.5, 128.4, 127.6, 127.3, 127.2, 126.6, 125.6, 125.1, 124.9, 124.7, 123.4, 121.1, 110.1, 72.7, 56.7, 56.3, 50.3, 43.3, 42.9, 39.9, 38.8, 37.4, 36.7, 32.9, 32.3, 32.1, 31.9, 26.0, 24.3, 21.2, 19.5, 18.3, 12.9, -4.5 (on observe seulement 44 des 45 résonances attendues); MS (CI, NH₃) m/z 660 (10, [M + NH₄]⁺), 511 (100).

### Synthèse du 3β-hydroxy-prégn-5-ène-21-(1-méthylpyrényl)-20-méthylidène (composé 7).

On ajoute 2,5 mL de TBAF (1M) dans du THF à une solution de composé **6** (300 mg, 466 µmol) dans 5 mL de THF sec et on agite la solution et on la chauffe à 65°C pendant 12h. Après refroidissement, on ajoute 100 mL d'eau et on extrait les produits organiques avec du dichlorométhane (3 x 50 mL). Les extraits organiques combinés sont rincés avec de la saumure, séchés sur du sulfate de sodium, filtrés, et évaporés. La purification par chromatographie flash sur silice G60 (0.040-0.063 mm), l'élution avec du CH₂Cl₂ dans de l'AcOEt (98/2) donnent 209 mg (85%) de composé 7 sous forme d'un solide blanc cassé ; mp 84°C; TLC (CH₂Cl₂/AcOEt 98:2) *R_{f}* 0.4; IR (KBr, cm⁻¹) 3364m, 2929s, 1636w, 1458w, 1434w, 1375w, 1182w, 1056m, 888m, 843s, 712w; ¹H RMN (CDCl₃) δ 0.52 (s, 3H), 0.65-2.25 (m, 32H), 2.3-2.6 (m, H), 3.27-3.44 (m, H), 4.83 (s, 1H), 4.99 (s, 1H), 5.24 (d, *J* = 4.5 Hz, 1H), 7.75-8.18 (m, 9H); ¹³C RMN (CDCl₃) δ 149.1, 140.8, 136.8, 131.5, 130.9, 129.8, 128.6, 127.5, 127.3, 127.2, 126.6, 125.8, 125.1, 124.9, 124.7, 123.4, 121.6, 110.1, 71.8, 56.6, 56.3, 50.2, 43.2, 42.3, 39.9, 38.8, 37.3, 36.6, 32.9, 32.3, 31.8, 31.7, 29.7, 26.0, 24.3, 21.1, 19.4, 12.9 (on observe seulement 38 des 39 résonances attendues); MS (CI, NH₃) m/z 546 (38, [M + NH4]⁺), 529 (89, [M + H]⁺), 217 (100).

### Chargement des méthyl-β-cyclodextrines par le Pyrène cholestérol (4) ou (7).

A 3ml d'une solution aqueuse de méthyl-β-cyclodextrines à 75mM portée à une température de 75°C, on ajoute progressivement 20µl d'une solution saturée de Pyrène cholestérol (4) ou (7) dissout à saturation dans la DMF (∼187,5mM soit 1mg/10µl de DMF) . Laisser agiter 30mn à 75°C puis laisser refroidir. La solution est filtrée sur filtre à 0,22µm et peut être conservée à 4°C plusieurs jours. Elle correspond à 1 Pyrène cholestérol (4) ou (7) pour 60 molécules de méthyl-β-cyclodextrines.

### Marquage des cellules

Des cellules à confluence immobilisées sur lamelle de silice de 22 par 22mm sont placées dans une boîte de pétri de 35mm de diamètre et recouvertes par 1,2 ml d'une solution filtrée à 0.22µm et composée par 15ml de milieu de base (DMEM à 1% de veau foetal) plus 1ml de solution de méthyl-β-cyclodextrines chargées en Pyrène cholestérol (4) ou (7). L'ensemble est laissé incuber à 37°C entre 1 et 15mn suivant le taux de marquage désiré. Puis le milieu est remplacé à 4 fois par 1,2 ml de PBS Ca++ Mg++.

Les mesures sont faites soit sur ces préparation ou après avoir remplacé le PBS par DMEM.

Les images sont réalisées sur un microscope axioplanII, de Zeiss grâce à un objectif à immersion d'huile de 63 (Plan-Neofluoar, NA=1,4) et une caméra CCD (C2400-75i ,Hamamatsu) équipée du système Argus 20 (Hamamatsu) qui soustrait le bruit de fond en directe sur la préparation correspondant au montage suivant : après avoir retourné la lamelle portant les cellules marquées sur une lame munie d'entretoises une chambre dont le volume est voisin de 120µl est ainsi réalisée et remplie de milieu DMEM. Les deux extrémités du montage sont scellées par collage sur les entretoises, les deux autres côtés restent ouverts pour permettre l'introduction d'additif extrinsèques : ligands etc.

Les spectres de fluorescence sont réalisés sur ces montages sur un spectrofluorimètre AMINCO 500. La longueur d'onde d'excitation est 335nm bp 5nm, le spectre d'émission étant enregistré après 370nm bp 4nm et avec une résolution de 0,25 nm par point.

### Résultat des mesures des spectres de fluorescence sur neuroblastome SH-SY5Y surexprimant ou n'exprimant pas HMOP et avec ou sans pyrène-cétone-cholestérol de formule (4)(figure 2)

Par comparaison des spectres en pointillé noir et continu gris qui correspondent au même tapis cellulaire de cellules SH-SY5Y surexprimant HMOP, on remarque qu'après ajout de l'agoniste DAMGO, alors que les spectres au niveau des bandes I3 et I4 sont inchangés, au contraire les bandes I1 ont des intensités différentes. Ceci indique, à cause de la diminution du spectre représenté en gris, une diminution de la polarité membranaire après ajout de DAMGO. Elle correspond à une augmentation de la quantité de phase 1o (c'est à dire une augmentation de l'épaisseur moyenne membranaire) induite par le récepteur à la suite d'ajout du DAMGO.

### REFERENCES

[1] Simons, K., and D. Toomre (2000). Nat. Rev. Mol. Cell Biol. 1:31-39.
[2] London, E., and D.A. Brown (2000). Biochim. Biophys. Acta 1508:182-195.
[3] Giacondi, M.C., V. Vie, E. Lesniewska, J.P. Goudonnet, and C. Le Grimellec (2000). J. Struct. Biol. 131:38-43.
[4] Simons, K., and E. Ikonen (1997). Nature 387:569-572.
[5] Ipsen, J.H., G. Karlstrom, O.G. Mouritsen, H. Wennerstrom, M.J. Zuckermann (1987). Biochim. Biophys. Acta 905:162-172.
[6] Sankaram, M.B. and T.E. Thompson (1990). 29:10676-84.
[7] Vist, M.R., J. H. Davis (1990). Biochemistry. 29:451-464.
[8] McMullen, T. P., McElhaney (1995). Biochim. Biophys. Acta. 1234:90-98.
[9] Srivastava, M., H. Ow, D. Larson, D. Holowka, U. Wiesner, W. Webb, B; Baird (2002). Biophys. J. 82:498a.
[10] Jacobson, K., C. Dietrich (1999). Trends Cell Biol. 9:87-91.
[11] Salamon Z, E. Varga, G. Tollin (1994). Biochemistry 33:13706.
[12] Salamon, Z., S. Cowell, E. Varga, H.I. Yamamura, V.J. Hruby, G. Tollin (2000). Biophys. J. 79:2463-2474.
[13] Dumas F., M.M. Sperotto, M..C. Lebrun, J.F. Tocanne, O.G. Mouritsen (1997). Biophys. J. 73:1940-1953.
[14] Dumas F., M.C. Lebrun, J.F. Tocanne (1999). FEBS Lett. 458:271-277.
[15] Lagane B., G. Gaibelet, E. Meilhoc, JM Masson, L. Cézanne, A. Lopez J. Biol. Chem. (2000). 327:33197-33200
[16] "De la distribution latérale du cholestérol dans les membranes biologiques à son rôle dans le contrôle de l'activité des protéines membranaires- cas du RCPGµ". B. Lagane, Thèse de l'Université P. Sabatier. Toulouse, Nov. 2001.
[17] Lagane B., S. Mazères, C. Le Grimellec, L. Cézanne, A Lopez, Biophys. Chem. (2002). 95 :7-22.
[18] L'Heureux, GP., M. Fragata (1987). J. Coll. Int. Scien. 117 :513-521.
[19] Mazères, S., B. Lagane, M. Welby, V. Trégou, A. Lopez (2001). Spectrochim. Acta A57:2297-2311.
[20] Mazères S., F. Rodriguez, J.F. Tocanne et A. Lopez (1996). Analusis 24:M20-M22.
[21] Daumas F., N. Destainville, C. Millot, A. Lopez, D. Dean, L. Salomé (2003). Biophys. J. 84:356-366
[22] Salomé L., F. Daumas, N. Destainville, C. Millot, A. Lopez, D. Dean (2002). Biophys. J. 82:40a
[23] Daumas F., M. Corbani, S. Ducasse, C. Millot, A. Lopez, L. Salomé (2002). Biophys. J. 82:216a
[24] Salomé L., J-L Cazeil, A Lopez, J F Tocanne (1998). Eur Biophys J. 27:391-402
[25] Cézanne L., S. Lecat, B. Lagane, C. Millot, JY.Vollmer, H. Matthes, JL.Galzi, A. Lopez (2004). J. Biol. Chem. 279:45057-45067.
[26] Ohtani, Y., T. Irie, K. Uekama, K. Fukunaga, and J. Pitha (1989) Eur J Biochem. 186 :17-22.

## Revendications

1. Composés ci-après dénommés pyrène-cholestérols, de formule générale (I) : 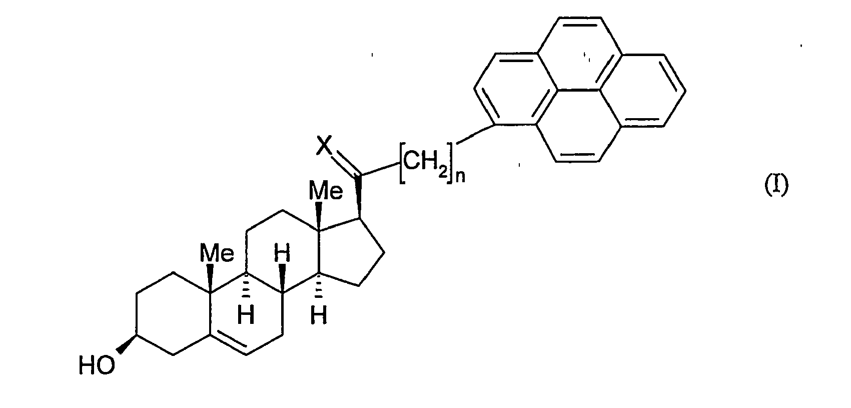 dans laquelle X représente un groupe CH₂ ou un atome d'oxygène et n est un nombre entier de 2 à 10, notamment de 2 à 6.

2. Composé selon la revendication 1, ci-après dénommé pyrène-cétone-cholestérol, de formule (4) suivante :

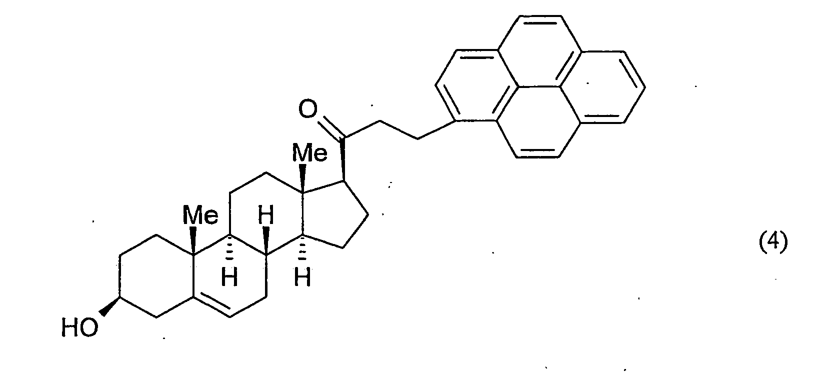

3. Composé selon la revendication 1, ci-après dénommé pyrène-méthylénique-cholestérol, de formule (7) suivante :

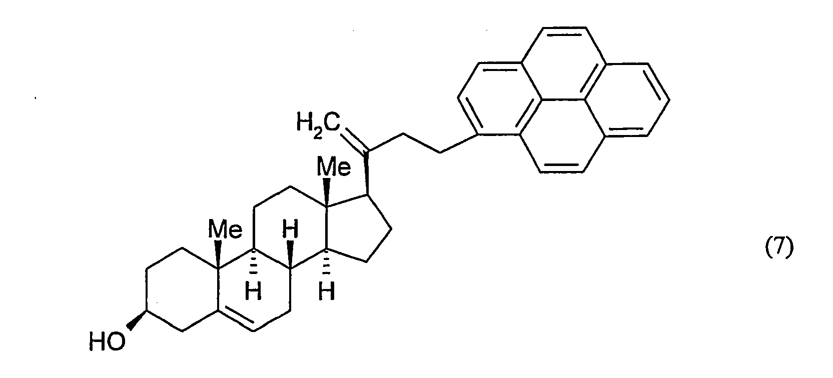

4. Utilisation d'un composé pyrène-cholestérol défini dans l'une des revendications 1 à 3, ou d'une combinaison de ces composés pyrène-cholestérol, pour l'étude de l'organisation fonctionnelle des membranes biologiques, le cas échéant en présence de stimuli extérieurs, tels que la température, la pression, la modulation par un agent extrinsèque de la concentration en un lipide contenu initialement dans lesdites membranes biologiques, ou l'ajout d'un produit déterminé.

5. Utilisation selon la revendication 4, pour la mise en oeuvre d'une méthode de détection, et, le cas échéant, de mesure de la polarité ou la perméabilité des membranes biologiques, la concentration d'un ou de plusieurs constituants de ces membranes, et de l'organisation spatiale et collective des constituants de ces membranes biologiques.

6. Utilisation selon l'une des revendications 4 ou 5, pour la mise en oeuvre d'une méthode de détection, et, le cas échéant, de mesure photophysique des effets de produits déterminés sur les membranes biologiques, ladite méthode étant appliquée :
- tau criblage de produits bioactifs susceptibles d'être des agonistes ou des antagonistes de récepteurs présents dans des membranes cellulaires ou à la surface de ces membranes, lesdits produits bioactifs étant susceptibles d'être utilisés pour la préparation de médicaments destinés au traitement de pathologies dans lesquelles interviennent lesdits récepteurs ;
- au diagnostic *in vitro* de pathologies à partir de cellules de patients à l'aide de produits déterminés utilisés en tant que marqueurs de ces pathologies, ou
- au contrôle de l'orientation de l'indication thérapeutique d'un médicament comprenant un produit bioactif tel que défini ci-dessus, par détermination in vitro de l'effet agoniste ou antagoniste de ce médicament sur les cellules du patient susceptibles d'être traité par ce médicament.

7. Utilisation selon l'une des revendications 4 à 6, **caractérisée en ce que** les membranes biologiques sont choisies parmi le groupe constitué de vésicules, de liposomes unilamellaires, de liposomes multilamellaires, de bicouches ou de multicouches lipidiques supportées, de membranes cellulaires extraites ou demembranes cellulaires de cellules vivantes.

8. Utilisation selon l'une des revendications 4 à 7, **caractérisée en ce que** les membranes cellulaires sont celles des cellules eucaryotes suivantes : CHO, HEK, NRK, AEB, AEH, SHY5Y, 3T3, cultures primaires de neurones, de fibrobastes, ou issues d'autres organes ou tissus.

9. Procédé pour l'étude de l'organisation fonctionnelle des membranes biologiques, et la mise en oeuvre d'une méthode de détection, et, le cas échéant, de mesure des effets de stimuli extérieurs, tels que la température, la pression, la modulation par un agent extrinsèque de la concentration en un lipide contenu initialement dans lesdites membranes biologiques, ou l'ajout d'un produit déterminé influençant l'organisation desdites membranes biologiques, **caractérisé en ce qu'**il comprend :
- une étape d'incorporation d'un composé selon l'une des revendications 1 à 3, ou d'une association de plusieurs de ces composés, dans lesdites membranes biologiques,
- le cas échéant l'application de stimuli extérieurs tels que définis dans la revendication 5, sur lesdites membranes,
- l'application sur lesdites membranes biologiques d'un rayonnement de longueur d'onde λₑₓ caractéristique de l'absorption du pyrène, λₑₓ étant compris entre 330 nm et 350 nm,
- la mesure de l'intensité du rayonnement de fluorescence émis par lesdites membranes biologiques à la longueur d'onde d'émission de 375 à 385 nm, caractéristique de la bande d'émission 1 du pyrène, cette intensité étant fonction de la polarité de la membrane,
- la mesure de l'intensité d'au moins un des deux rayonnements de fluorescence émis par lesdites membranes biologique entre 390 nm et 425 nm, caractéristiques des bandes d'émission 3 et 4 du pyrène, cette intensité étant fonction de la quantité de pyrène, et donc de composés selon l'une des revendications 1 à 3, dans lesdites membranes,
- la détermination du rapport entre l'intensité de la bande 1 et celle de la bande 3 et/ou 4, à savoir des rapports I₁/I₃ et/ou I₁/I₄,
- la corrélation entre le rapport mesuré lors de l'étape précédente, et le niveau de polarité desdites membranes qui est fonction de leur épaisseur, donnant ainsi une indication sur l'organisation fonctionnelle de ces membranes en présence ou non de stimuli extérieurs.

10. Procédé selon la revendication 9, appliqué
au criblage de produits bioactifs susceptibles d'être des agonistes ou des antagonistes de récepteurs présents dans des membranes cellulaires ou partiellement insérés dans ces membranes, lesdits produits bioactifs étant susceptibles d'être utilisés pour la préparation de médicaments destinés au traitement de pathologies dans lesquelles interviennent lesdits récepteurs, ou
au contrôle de l'orientation de l'indication thérapeutique d'un médicament comprenant un produit bioactif tel que défini ci-dessus, par détermination in vitro de l'effet agoniste ou antagoniste de ce médicament sur les cellules du patient susceptibles d'être traité par ce médicament,
ledit procédé comprenant :
- une étape d'incorporation d'un composé selon l'une des revendications 1 à 3, ou d'une association de plusieurs de ces composés, dans lesdites membranes biologiques,
- la mise en présence desdites membranes avec un produit bioactif, ou une banque de produits bioactifs, dans des conditions permettant la liaison de ces produits avec les récepteurs membranaires,
- le cas échéant, une étape de rinçage desdites membranes pour éliminer tout produit bioactif qui ne se serait pas susceptible de se lier avec un récepteur membranaire,
- l'application sur lesdites membranes biologiques d'un rayonnement de longueur d'onde λₑₓ caractéristique de l'absorption du pyrène, λₑₓ étant compris entre 330 nm et 350 nm,
- la mesure de l'intensité des bandes 1, 3, et/ou 4, et la détermination des rapports I₁/I₃ et/ou I₁/L₄,
- la comparaison des rapports I₁/I₃ et/ou I₁/I₄, obtenus à l'étape précédente avec les rapports I'₁/I'₃ et/ou I'₁/I'₄, mesurés en l'absence de produits bioactifs, ce qui permet de déterminer la nature agoniste ou antagoniste ou antagoniste inverse du ou des produits bioactifs testés, un produit agoniste étant **caractérisé par** un rapport I₁/I₃ et/ou I₁/I₄ respectivement supérieur ou inférieur au rapport I₁/I₃ et/ou I₁/I₄ suivant le type desdits récepteurs, un produit antagoniste étant **caractérisé par** un rapport I₁/I₃ et/ou I₁/I₄ équivalent respectivement au rapport I'₁/I'₃ et/ou I'₁/I'₄, un produit antagoniste inverse étant **caractérisé par** un rapport I₁/I₃ et/ou I₁/I₄ respectivement inférieur ou supérieur au rapport I'₁/I'₃ et/ou I'₁/I'₄ soit un effet inverse à celui d'un produit agoniste.

11. Procédé selon la revendication 9 ou 10, **caractérisé en ce qu'**il est effectué in vitro sur des cellules eucaryotes, et comprend une étape d'incorporation d'un composé selon l'une des revendications 1 à 3, ou d'une association de plusieurs de ces composés, dans les membranes de ces cellules.

12. Procédé selon l'une des revendications 9 à 11, **caractérisé en ce que** l'étape d'incorporation d'un composé selon l'une des revendications 1 à 3, ou de l'association de plusieurs de ces composés, dans lesdites membranes s'effectue au moyen d'un transporteur destiné à échanger le cholestérol, un phospholipide, naturellement présents dans lesdites membranes, avec ledit composé pyrène-cholestérol ou ladite combinaison.

13. Procédé selon l'une des revendications 9 à 12, **caractérisé en ce que** le transporteur est une méthyl-β-cyclodextrine, ou une α-, β-, γ-cyclodextrine.

14. Procédé selon l'une des revendications 9 à 13, **caractérisé en ce que** la proportion de composé selon l'une des revendications 1 à 3, ou de l'association de plusieurs de ces composés, incorporée dans lesdites membranes représente environ 0,1 à 2 % de la concentration surfacique des lipides présents dans la membrane biologiques.

15. Composition **caractérisée en ce qu'**elle comprend un composé selon l'une des revendications 1 à 3, ou une association de plusieurs de ces composés, en mélange avec un transporteur destiné à échanger le cholestérol ou un phospholipide, naturellement présents dans lesdites membranes avec ledit composé pyrène-cholestérol ou ladite combinaison

16. Composition selon la revendication 15, **caractérisée en ce que** le transporteur est une méthyl-β-cyclodextrine, ou une α-, β-, γ-cyclodextrine.

17. Utilisation d'une composition selon la revendication 15 ou 16, dans l'étape d'incorporation d'un composé selon l'une des revendications 1 à 3, ou d'une association de plusieurs de ces composés, dans lesdites membranes biologiques, lors de la mise en oeuvre d'un procédé selon l'une des revendications 9 à 14.

18. Procédé de synthèse des composés de formule (I) selon l'une des revendications 1 à 3, **caractérisé en ce qu'**il comprend les étapes suivantes :
a) la réaction de la prégnénolone de formule (a) et du dérivé du pyrène de formule (b) dans laquelle n est tel que défini dans la revendication 1, en présence de EtOK, EtOH, dans CH₂Cl₂ à une température entre 5°C et 20°C et pendant une durée comprise entre 10 et 72 heures, ce qui conduit à l'obtention du composé de formule (1) suivant : 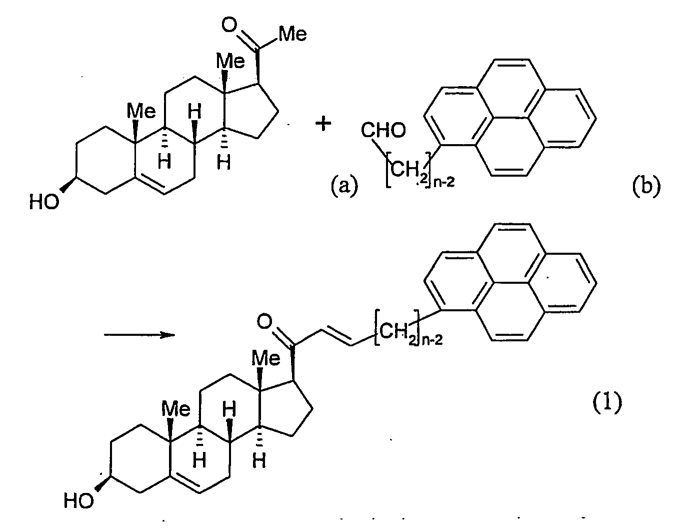
b) la réaction du composé de formule (1) obtenu à l'étape précédente en présence de 1,5 équivalents de Ac₂O, de 10 mol % de DMAP, de 1 équivalent de Et₃N, dans CH₂Cl₂ pendant 3 heures, ce qui conduit à l'obtention du composé de formule (2) suivant : 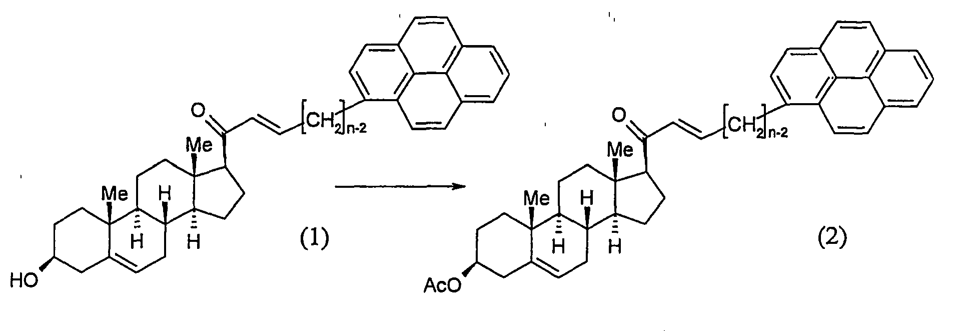
c) la réaction du composé de formule (2) obtenu à l'étape précédente en présence de 2 équivalents de TiCl₄, de 1,5 équivalents de Et₃SiH, dans CH₂Cl₂ pendant 45 minutes à température ambiante, ce qui conduit à l'obtention du composé de formule (3) suivant : 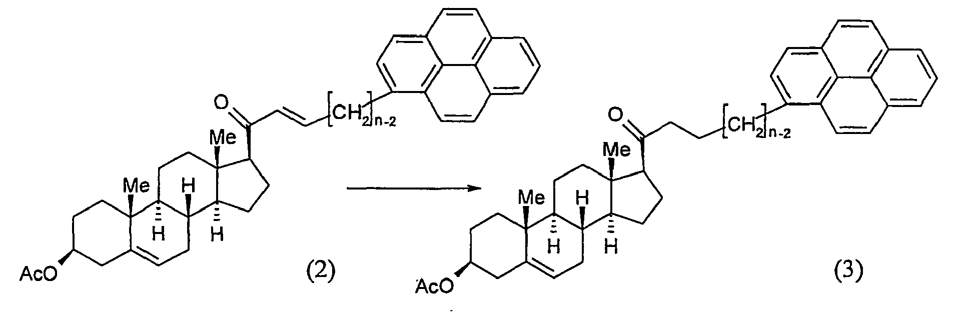
d) la réaction du composé de formule (3) obtenu à l'étape précédente en présence de MeONa, dans CH₂Cl₂ pendant 4 heures à température ambiante, conduit au composé de formule (4) suivant : 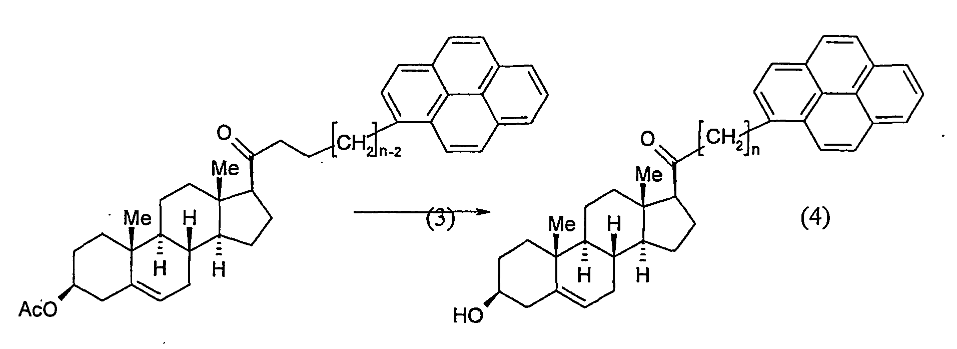
e) le cas échéant, la réaction du composé de formule (4) obtenu à l'étape précédente en présence de TBDMSCl, d'imidazole, dans CH₂Cl₂ pendant 12 heures à température ambiante, ce qui conduit à l'obtention du composé de formule (5) suivant: 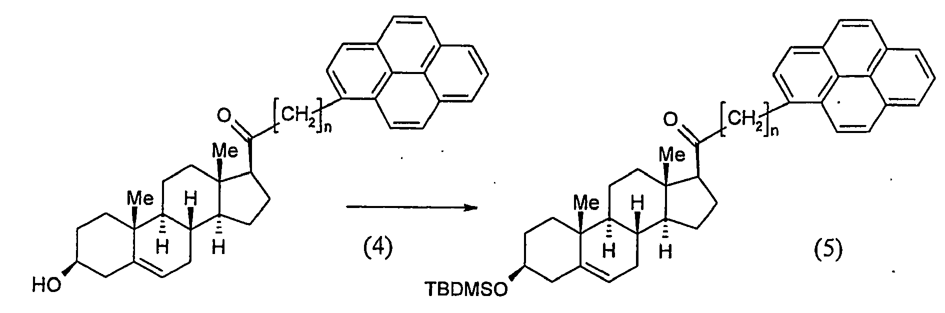
f) la réaction du composé de formule (5) obtenu à l'étape précédente en présence de 2 équivalents de CH₂=PPh₃, dans un mélange THF/DMSO, pendant 12 heures à 75°C, ce qui conduit à l'obtention du composé de formule (6) suivant : 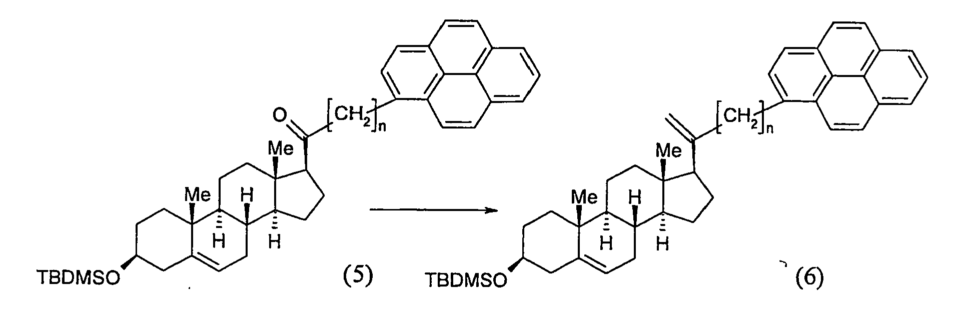
g) la réaction du composé de formule (6) obtenu à l'étape précédente en présence de 5 équivalents de TBAF, dans le THF, pendant 12 heures à 65°C, conduit au composé de formule (7) suivant :

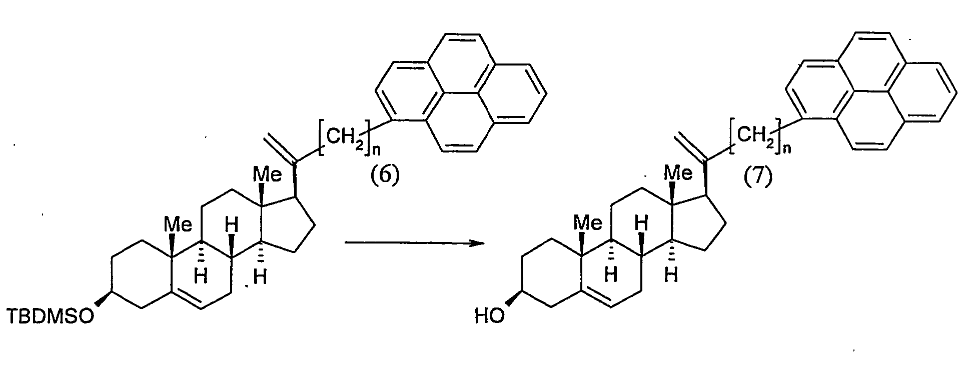

## Claims

1. Compounds hereafter called pyrene-cholesterols, of general formula (I): 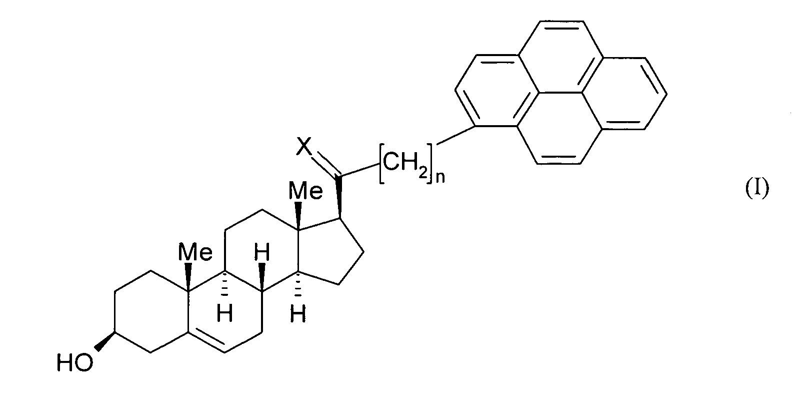 in which X represents a CH₂ group or an oxygen atom and n is an integer from 2 to 10, in particular from 2 to 6.

2. Compound according to claim 1, hereafter called pyrene-ketone-cholesterol, of following formula (4):

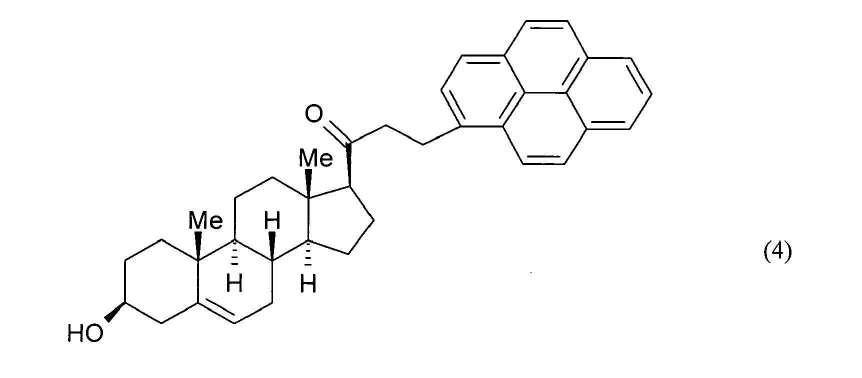

3. Compound according to claim 1, hereafter called pyrene-methylene-cholesterol, of following formula (7):

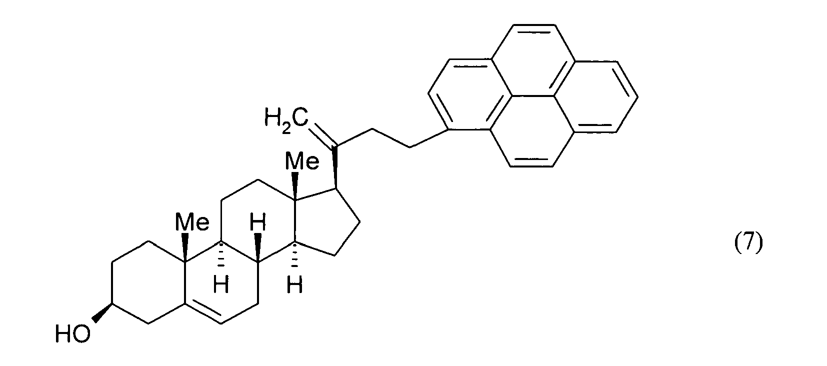

4. Use of a pyrene-cholesterol compound defined in one of claims 1 to 3, or a combination of these pyrene-cholesterol compounds, for the study of the functional organization of biological membranes, if appropriate in the presence of external stimuli, such as temperature, pressure, modulation by an extrinsic agent of the lipid concentration initially contained in said biological membranes, or the addition of a given product.

5. Use according to claim 4, for implementing a method of detection, and, if appropriate, measurement of the polarity or the permeability of biological membranes, the concentration of one or more constituents of these membranes, and the spatial and collective organization of the constituents of these biological membranes.

6. Use according to one of claims 4 or 5, for implementing a method of detection, and, if appropriate, photophysical measurement of the effects of given products on biological membranes, said method being applied to:
- screening bioactive products capable of being agonists or antagonists of receptors present in cell membranes or on the surface of these membranes, said bioactive products capable of being used for the preparation of medicaments intended for the treatment of pathologies involving said receptors;
- *in vitro* diagnosis of pathologies from cells of patients using given products used as markers of these pathologies, or
- monitoring the orientation of the therapeutic indication of a medicament comprising a bioactive product as defined above, by determination *in vitro* of the agonist or antagonist effect of this medicament on the cells of the patient capable of being treated with this medicament.

7. Use according to one of claims 4 to 6, **characterized in that** the biological membranes are chosen from the group constituted by vesicles, unilamellar liposomes, multilamellar liposomes, supported lipid bilayers or multilayers, extracted cell membranes or cell membranes of living cells.

8. Use according to one of claims 4 to 7, **characterized in that** the cell membranes are those of the following eukaryotic cells: CHO, HEK, NRK, AEB, AEH, SHY5Y, 3T3, primary cultures of neurones, fibroblasts, or originating from other organs or tissues.

9. Method for the study of the functional organization of biological membranes, and the use of a method of detection, and, if appropriate, measurement of the effects of external stimuli, such as temperature, pressure, the modulation by an extrinsic agent of the lipid concentration initially contained in said biological membranes, or the addition of a given product influencing the organization of said biological membranes, **characterized in that** it comprises:
- a step of incorporation of a compound according to one of claims 1 to 3, or a combination of several of these compounds, into said biological membranes,
- if appropriate, the application of external stimuli as defined in claim 5, to said membranes,
- the application to said biological membranes of a radiation of wavelength λₑₓ characteristic of the absorption of pyrene , λₑₓ being comprised between 330 nm and 350 nm,
- the measurement of the intensity of the fluorescence radiation emitted by said biological membranes at the emission wavelength of 375 to 385 nm, characteristic of pyrene emission band 1, this intensity being a function of the polarity of the membrane,
- the measurement of the intensity of at least one of the two fluorescence radiations emitted by said biological membranes between 390 nm and 425 nm, characteristic of pyrene emission bands 3 and 4, this intensity being a function of the quantity of pyrene, and therefore of compounds according to one of claims 1 to 3, in said membranes,
- the determination of the ratio between the intensity of band 1 and that of band 3 and/or 4, namely of the ratios I₁/I₃ and/or I₁/I₄,
- the correlation between the ratio measured during the previous step, and the level of polarity of said membranes which is a function of their thickness, thus giving an indication of the functional organization of these membranes in the presence or absence of external stimuli.

10. Method according to claim 9, applied to
the screening of bioactive products capable of being agonists or antagonists of receptors present in cell membranes or partially inserted into these membranes, said bioactive products being capable of being used for the preparation of medicaments intended for the treatment of pathologies in which said receptors are involved, or
the monitoring of the direction of the therapeutic indication of a medicament comprising a bioactive product as defined above, by *in vitro* determination of the agonist or antagonist effect of said medicament on the cells of the patient capable of being treated with this medicament,
said method comprising:
- a step of incorporation of a compound according to one of claims 1 to 3, or a combination of several of these compounds, into said biological membranes,
- the placing of said membranes in the presence of a bioactive product, or a bank of bioactive products, under conditions allowing these products to bind with the membrane receptors,
- if appropriate, a step of rinsing said membranes to remove any bioactive product which is not capable of bonding with a membrane receptor,
- the application to said biological membranes of a radiation of wavelength of λₑₓ characteristic of the absorption of pyrene , λₑₓ being comprised between 330 nm and 350 nm,
- the measurement of the intensity of bands 1, 3, and/or 4, and the determination of the ratios I₁/I₃ and/or I₁/I₄,
- the comparison of the ratios I₁/I₃ and/or I₁/I₄, obtained in the previous step with the ratios I'₁/I'₃ and/or I'₁/I'₄, measured in the absence of bioactive products, which allows the agonist or antagonist or inverse antagonist nature of the bioactive products tested to be determined, an agonist product being **characterized by** a ratio I₁/I₃ and/or I₁/I₄ respectively greater than or less than the ratio I'₁/I'₃ and/or I'₁/I'₄ according to the type of said receptors, an antagonist product being **characterized by** a ratio I₁/I₃ and/or I₁/I₄ equivalent respectively to the ratio I'₁/I'₃ and/or I'₁/I'₄, an inverse antagonist product being **characterized by** a ratio I₁/I₃ and/or I₁/I₄ respectively less than or greater than the ratio I'₁/I'₃ and/or I'₁/I'₄ i.e. an inverse effect to that of an agonist product.

11. Method according to claim 9 or 10, **characterized in that** it is carried out in vitro on eukaryotic cells, and comprises a stage of incorporation of a compound according to one of claims 1 to 3, or a combination of several of these compounds, into the membranes of these cells.

12. Method according to one of claims 9 to 11, **characterized in that** the stage of incorporation of a compound according to one of claims 1 to 3, or the combination of several of these compounds, into said membranes is carried out using a transporter intended to exchange cholesterol, a phospholipid, naturally present in said membranes, with said pyrene-cholesterol compound or said combination.

13. Method according to one of claims 9 to 12, **characterized in that** the transporter is a methyl-β-cyclodextrin, or a α-, β-, γ-cyclodextrin.

14. Method according to one of claims 9 to 13, **characterized in that** the proportion of compound according to one of claims 1 to 3, or the combination of several of these compounds, incorporated into said membranes represents approximately 0.1 to 2 % of the surface concentration of the lipids present in the biological membrane .

15. Composition **characterized in that** it comprises a compound according to one of claims 1 to 3, or a combination of several of these compounds, in a mixture with a transporter intended to exchange cholesterol or a phospholipid, naturally present in said membranes with said pyrene-cholesterol compound or said combination.

16. Composition according to claim 15, **characterized in that** the transporter is a methyl-β-cyclodextrin, or a α-, β-, γ-cyclodextrin.

17. Use of a composition according to claim 15 or 16, in the stage of incorporation of a compound according to one of claims 1 to 3, or a combination of several of these compounds, into said biological membranes, during the implementation of a method according to one of claims 9 to 14.

18. Synthesis method for the compounds of formula (I) according to one of claims 1 to 3, **characterized in that** it comprises the following steps:
a) reacting the pregnenolone of formula (a) and the pyrene derivative of formula (b) in which n is as defined in claim 1, in the presence of EtOK, EtOH, in CH₂Cl₂ at a temperature between 5°C and 20°C and for a duration comprised between 10 and 72 hours, which produces the following compound of formula (1): 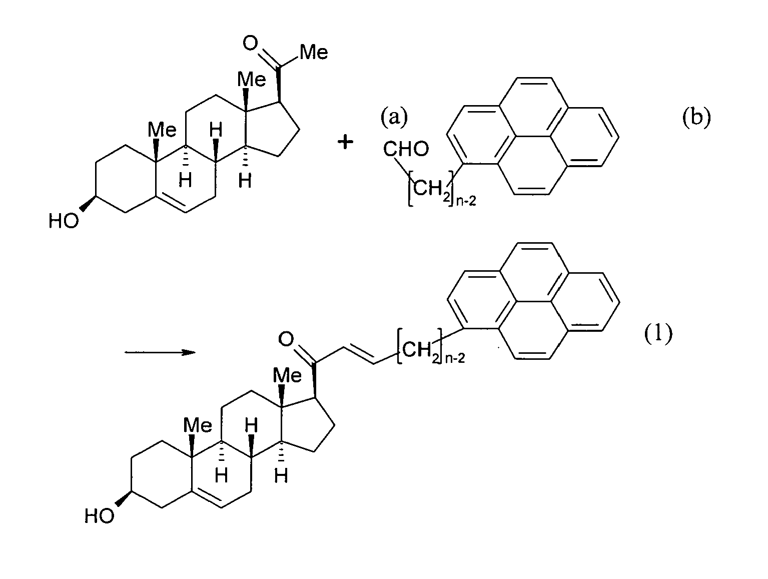
b) reacting the compound of formula (1) obtained in the previous step in the presence of 1.5 equivalents of Ac₂O, 10 mol % of DMAP, 1 equivalent of Et₃N, in CH₂Cl₂ for 3 hours, which produces the following compound of formula (2): 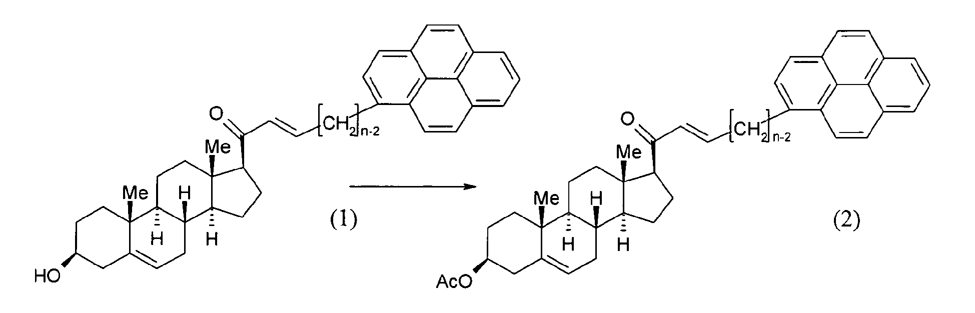
c) reacting the compound of formula (2) obtained in the previous step in the presence of 2 equivalents of TiCl₄, 1.5 equivalents of Et₃SiH, in CH₂Cl₂ for 45 minutes at ambient temperature, which produces the following compound of formula (3) : 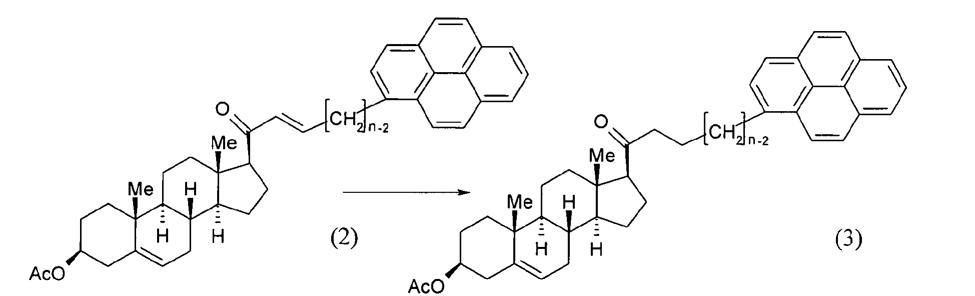
d) reacting the compound of formula (3) obtained in the previous step in the presence of MeONa, in CH₂Cl₂ for 4 hours at ambient temperature, leads to the following compound of formula (4): 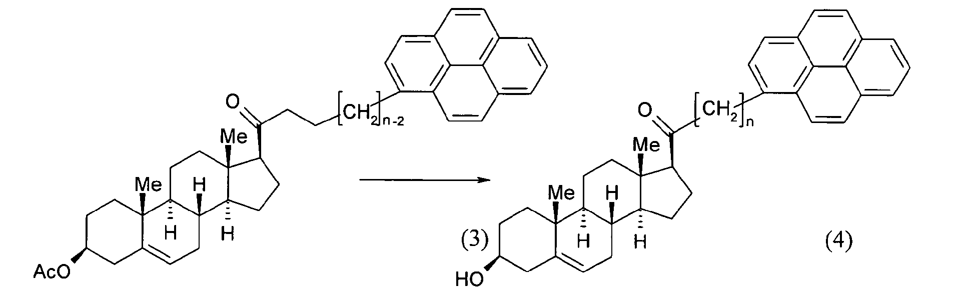
e) if appropriate, reacting the compound of formula (4) obtained in the previous step in the presence of TBDMSCI, imidazole, CH₂Cl₂ for 12 hours at ambient temperature, which produces the following compound of formula (5): 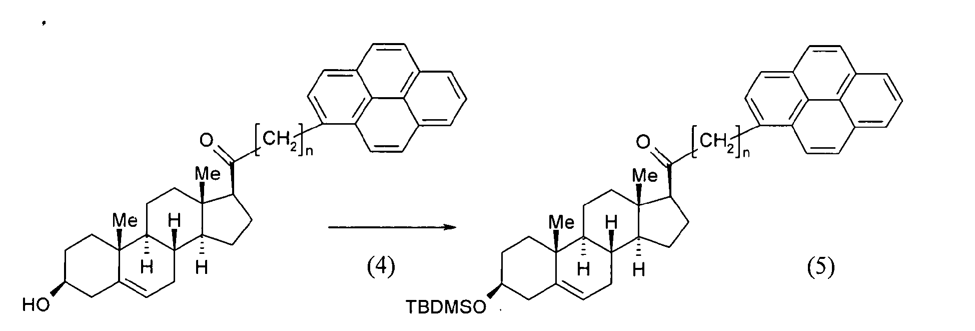
f) reacting the compound of formula (5) obtained in the previous step in the presence of 2 equivalents of CH₂=PPh₃, in a THF/DMSO mixture, for 12 hours at 75°C, which produces the following compound of formula (6): 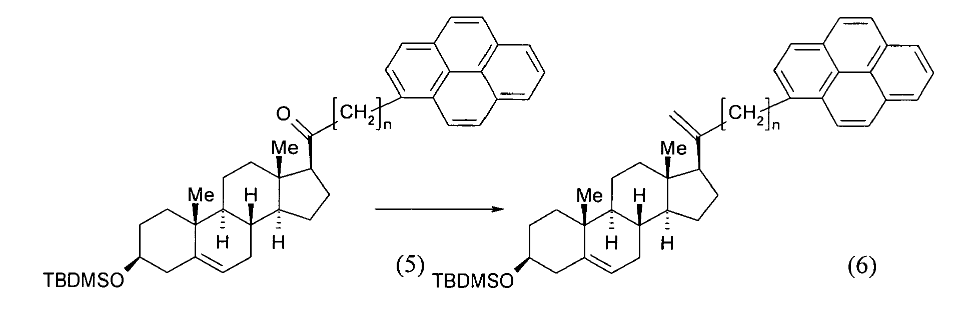
g) reacting the compound of formula (6) obtained in the previous step in the presence of 5 equivalents of TBAF, in THF, for 12 hours at 65°C, produces the following compound of formula (7):

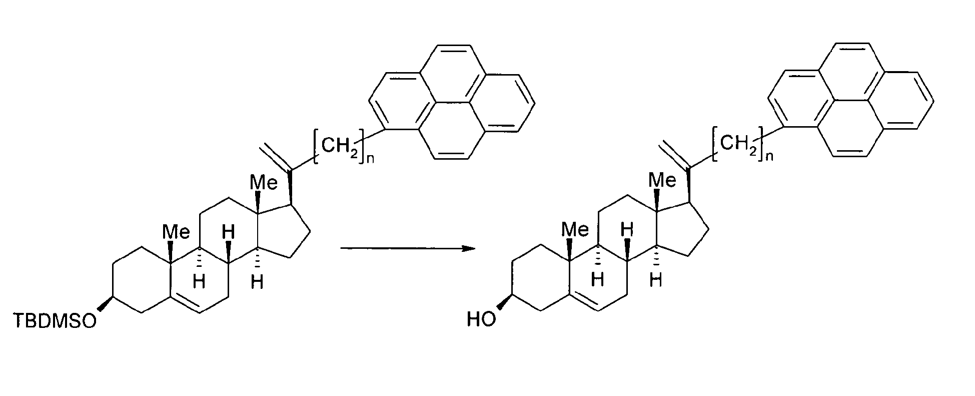

## Patentansprüche

1. Verbindungen, die hierunten Pyrene-Cholesterine genannt werden, mit der allgemeinen Formel (I) : 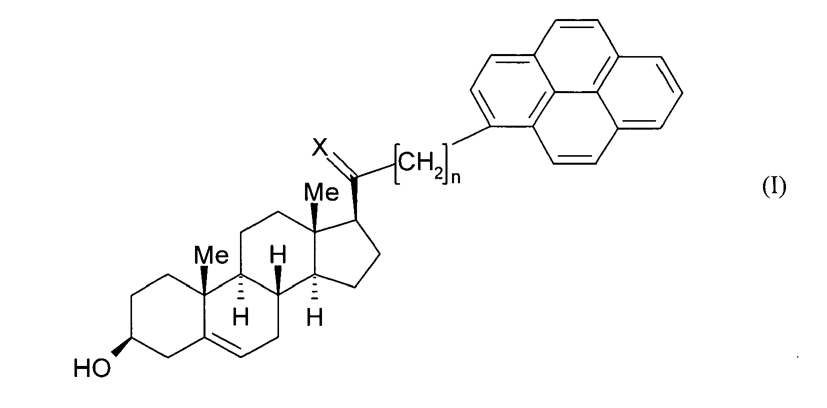 worin X eine CH₂-Gruppe oder ein Sauerstoffatom bedeutet, und n eine ganze Zahl von 2 bis 10, insbesondere von 2 bis 6 ist.

2. Verbindung nach Anspruch 1, die hierunten Pyren-Keton-Cholesterin genannt wird, mit der folgenden Formel (4) :

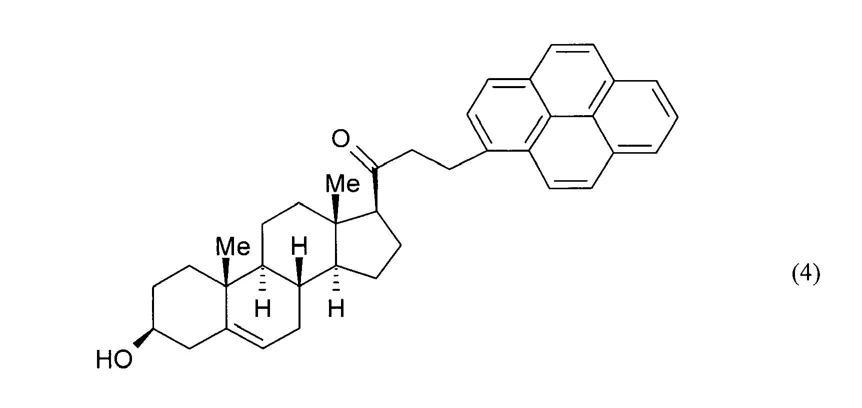

3. Verbindung nach Anspruch 1, die hierunten Pyren-methylenisch-Cholesterin genannt wird, mit der folgenden Formel (7) :

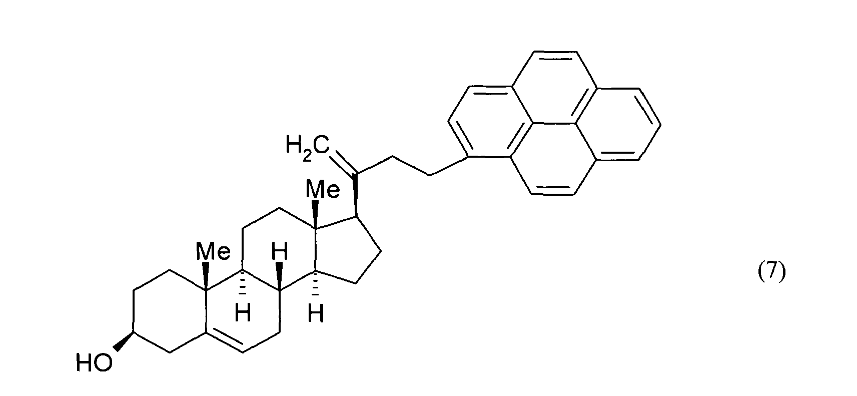

4. Verwendung einer Pyren-Cholesterin Verbindung wie in Ansprüche 1 - 3 definiert, oder einer Kombination von diesen Pyren-Cholesterin Verbindungen, für die Erforschung der funktionellen Organisation der biologischen Membranen, gegebenenfalls in Anwesenheit von äußeren Stimuli, wie z. B. Temperatur, Druck, Modulation durch ein äußerliches Mittel der Konzentration in einem Lipid, das ursprünglich in den genannten biologischen Membranen beinhaltet ist, oder die Zufügung eines bestimmten Produkts.

5. Verwendung nach Anspruch 4, für die Einführung einer Feststellungsmethode, und gegebenenfalls für die Messung der Polarität oder der Permeabilität der biologischen Membranen, der Konzentration einer oder mehreren Komponenten dieser Membranen, und der räumlichen und gemeinsamen Organisation der Komponenten dieser biologischen Membranen.

6. Verwendung nach irgendwelcher Anspruch 4 oder 5, für die Einführung einer Feststellungsmethode, und gegebenenfalls für die photophysische Messung der Wirkung von bestimmten Produkten auf die biologischen Membranen, worin die genannte Methode auf folgende Maßnahmen appliziert wird :
- Sieben von bioaktiven Produkten, die dazu fähig sind, Agonisten oder Antagonisten von Rezeptoren zu sein, die in Zellmembranen oder auf die Oberfläche dieser Membranen anwesend sind, worin diese bioaktiven Produkte für die Herstellung von Arzneimitteln für die Behandlung von Pathologien, worin die genannten Rezeptoren eine Rolle spielen benutzt werden können ;
- *In vitro* Diagnose von Pathologien aus Zellen von Patienten, und zwar mit bestimmten Produkten, die als Marker dieser Pathologien benutzt werden, oder
- Kontrolle der Orientierung der therapeutischen Indikation eines Arzneimittels, das ein wie oben definiertes bioaktives Produkt enthält, und zwar durch die in vitro Bestimmung der agonisten oder antagonisten Wirkung dieses Arzneimittels auf die Zellen des Patienten, worin diese Zellen fähig sind, mit diesem Arzneimittel behandelt zu werden.

7. Verwendung nach irgendwelcher Anspruch 4 - 6, **dadurch gekennzeichnet, daß** die biologischen Membranen unter der Gruppe gewählt werden, die aus Vesikulen, unilamellaren Liposomen, multilamellaren Liposomen, lipidischen Zweischichten oder Mehrschichten auf Stütze oder extrahierten Zellmembranen oder Zellmembranen aus lebenden Zellen gebildet wird.

8. Verwendung nach irgendwelcher Anspruch 4 - 7, **dadurch gekennzeichnet, daß** die Zellmembranen die der folgenden eukaryotischen Zellen sind : CHO, HEK, NRK, AEB, AEH, SHY5Y, 3T3, Primärkulturen von Neuronen oder Fibroblasten, oder aus anderen Organen oder Geweben.

9. Verfahren für die Erforschung der funktionellen Organisation von biologischen Membranen, und die Einführung einer Bestimmungsmethode, und gegebenenfalls die Messung der Wirkung von äußerlichen Stimuli, wie Temperatur, Druck, Modulation, durch ein äußerliches Mittel, der Konzentration in einem Lipid, das ursprünglich in den genannten biologischen Membranen beinhaltet ist, oder die Zuführung eines bestimmten Produkt, daß die Organisation der genannten biologischen Membranen beeinflußt, **dadurch gekennzeichnet, daß** es die folgenden Schritten einschließt :
- die Vermengung einer Verbindung nach irgendwelcher Anspruch 1 - 3, oder die Vereinigung von mehreren dieser Verbindungen in den obengenannten biologischen Membranen,
- gegebenenfalls, die Verwendung von äußerlichen Stimuli wie in Anspruch 5 definierte, auf die genannten Membranen,
- die Verwendung, auf die genannten biologischen Membranen, einer Strahlung mit einer Wellenlänge λₑₓ , die charakteristisch für die Absorption des Pyrens ist, worin λₑₓ zwischen 330 nm und 350 nm liegt,
- die Messung der Intensität der Fluoreszenzstrahlung, die von den genannten biologischen Membranen ausgestrahlt wird, und zwar bei der Sendungswellenlänge von 375 bis 385 nm, die charakteristisch für die Sendungsbandbreite 1 des Pyrens ist, worin diese Intensität von der Polarität der Membran abhängt,
- die Messung der Intensität von zumindest einer der beiden Fluoreszenzsstrahlungen, die von den genannten biologischen Membranen ausgestrahlt werden, und zwar zwischen 390 nm und 425 nm, worin diese Werte charakteristisch für die Sendungsbandbreiten 3 und 4 des Pyrens sind, und worin diese Intensität von der Menge des anwesenden Pyrens, und deshalb der Verbindungen nach irgendwelcher Anspruch 1 - 3, in den genannten Membranen abhängt,
- die Bestimmung des Verhältnisse zwischen der Intensität des Sendungsbands 1 und der des Bands 3 und/oder 4, das heißt der Verhältnisse I₁/I₃ und/oder I₁/I₄,
- die Beziehung zwischen dem gemessenen Verhältnis des vorigen Schrittes, und dem Polaritätsniveau der genannten Membranen, das von ihrer Dicke abhängt, die also einen Hinweis über die funktionnelle Organisation dieser Membrane in An- oder Abwesenheit von äußerlichen Stimuli angibt.

10. Verfahren nach Anspruch 9, das auf das Sieben von bioaktiven Produkten angewendet wird, worin diese bioaktiven Produkte fähig sind, Agonisten oder Antagonisten der in Zellmembranen anwesenden, oder teilweise in diesen Membranen inserierten Rezeptoren zu sein, und worin diese bioaktiven Produkte für die Herstellung von Arzneimitteln für die Behandlung von Pathologien, worin diese Rezeptoren eine Rolle spielen, benutzt werden können, oder
auf die Kontrolle der Orientierung der therapeutischen Indikation eines Arzneimittels, das ein wie oben definiertes bioaktives Produkt enthält, duch die in vitro Bestimmung der agonisten oder antagonisten Wirkung diess Arzneimittels auf die Zellen eines Patienten, worin dieser Patient mit diesem Arzneimittel behandelt werden kann,
worin dieses Verfahren folgende Schritte beinhaltet :
- die Vermengung einer Verbindung nach irgendwelcher Anspruch 1 - 3, oder die Vereinigung von mehreren dieser Verbindungen in den obengenannten biologischen Membranen,
- die Gegenüberstellung von den genannten Membranen mit einem bioaktiven Produkt, oder mit einer Bank von bioaktiven Produkten, und zwar unter solchen Bedingungen, die die Bindung von solchen Produkten mit den Membranrezeptoren erlauben,
- gegebenenfalls einen Ausspülungsschritt von den genannten Membranen, um irgendwelche bioaktive Produkte, die sich mit einem Membranrezeptor nicht binden könnten, zu beseitigen,
- die Verwendung, auf die genannten biologischen Membranen, einer Strahlung mit einer Wellenlänge λₑₓ , die charakteristisch für die Absorption des Pyrens ist, worin λₑₓ zwischen 330 nm und 350 nm liegt,
- die Messung der Intensität der Sendungsbänder 1, 3, und/oder 4, und die Bestimmung der Verhältnisse I₁/I₃ und/oder I₁/I₄,
- der Vergleich der Verhältnisse I₁/I₃ und/oder I₁/I₄, wie im vorigen Schritt mit den Verhältnissen I'₁/I'₃ und/oder I'₁/I'₄ erhaltenen, und wie in Abwesenheit von bioaktiven Produkten gemessen, was die Bestimmung des agonisten oder antagonisten, oder gegenantagonisten Wesen von dem oder von den getesteten Produkten erläubt, worin ein agonistisches Produkt **dadurch gekennzeichnet ist, daß** das Verhältnis I₁/I₃ und/oder I₁/I₄ jeweils höher oder niedriger als das Verhältnis I'₁/I'₃ und/oder I'₁/I'₄ nach der Art von den gesagten Rezeptoren ist, und worin ein antagonistisches Produkt **dadurch gekennzeichnet ist, daß** das Verhältnis I₁/I₃ und/oder I₁/I₄ jeweils dem Verhältnis I'₁/I'₃ und/oder I'₁/I'₄ entspricht, und worin ein gegenantagonistisches Produkt **dadurch gekennzeichnet ist, daß** das Verhältnis I₁/I₃ und/oder I₁/I₄ jeweils niedriger oder höher als das Verhältnis I'₁/I'₃ und/oder I'₁/I'₄ ist, also eine Wirkung, die zur Wirkung eines agonistischen Produktes entgegengesetzt ist.

11. Verfahren nach Anspruch 9 oder 10, **dadurch gekennzeichnet, daß** es in vitro auf eukaryontische Zellen angewendet wird, und daß es einen Vermengungsschritt von einer Verbindung nach irgendwelcher Anspruch 1 - 3, oder einen Vereinigunsschritt von mehreren dieser Verbindungen in den Membranen dieser Zellen einschließt.

12. Verfahren nach irgendwelcher Anspruch 9 - 11, **dadurch gekennzeichnet, daß** der Vermengungsschritt einer Verbindung nach irgendwelcher Anspruch 1 - 3, oder daß der Vereinigunsschritt von mehreren dieser Verbindungen in den genannten Membranen mit einem Träger für den Austausch von Cholesterin und Phospholipid, die in den genannten Membranen natürlich anwesend sind, gegen die gesagte Pyren-Cholesterin-Verbindung oder die gesagte Vereinigung durchgeführt wird.

13. Verfahren nach irgendwelcher Anspruch 9 - 12, **dadurch gekennzeichnet, daß** der Träger eine Methyl-β-Zyklodextrin, oder eine α-, β-, γ-Zyklodextrin ist.

14. Verfahren nach irgendwelcher Anspruch 9 - 13, **dadurch gekennzeichnet, daß** das Verhältnis der Verbindung nach irgendwelcher Anspruch 1 - 3, oder der Vereinigung von mehreren dieser Verbindungen, wie in den gesagten Membranen eingerührt, ungefähr 0,1 bis 2 % der Oberflächenkonzentration der anwesenden Lipiden in dem biologischen Membran darstellt.

15. Eine Zusammenstellung, die **dadurch gekennzeichnet ist, daß** sie eine Verbindung nach irgendwelcher Anspruch 1 - 3, oder eine Vereinigung von mehreren dieser Verbindungen einschließt, und zwar als Mischung mit einem Träger für den Austausch von Cholesterin oder Phospholipid, die in den genannten Membranen natürlich anwesend sind, gegen die gesagte Pyren-Cholesterin-Verbindung oder die gesagte Vereinigung.

16. Eine Zusammenstellung nach Anspruch 15, **dadurch gekennzeichnet ist, daß** der Träger eine Methyl-β-Zyklodextrin oder eine α-, β-, γ-Zyklodextrin ist.

17. Verwendung einer Zusammenstellung nach Anspruch 15 oder 16, im Vermengunsschritt einer Verbindung nach irgendwelcher Anspruch 1 - 3, oder einer Vereinigung von mehreren dieser Verbindungen, in den genannten biologischen Membranen, während der Ausführung eines Verfahrens nach den Ansprüchen 9 - 14.

18. Verfahren für die Synthese der Verbindungen der Formel (I) nach irgendwelcher Anspruch 1 - 3, **dadurch gekennzeichnet, daß** es die folgenden Schritte einschließt :
a) die Reaktion der Pregnenolon der Formel (a) und des Pyrenderivats der Formel (b), worin n wie in Anspruch 1 definiert ist, in Anwesenheit von EtOK, EtOH, in CH₂Cl₂ bei einer Temperatur zwischen 5°C - 20°C und während einer Dauer von 10 - 72 Stunden, was zum Erhalten der folgenden Verbindung der Formel (1) führt : 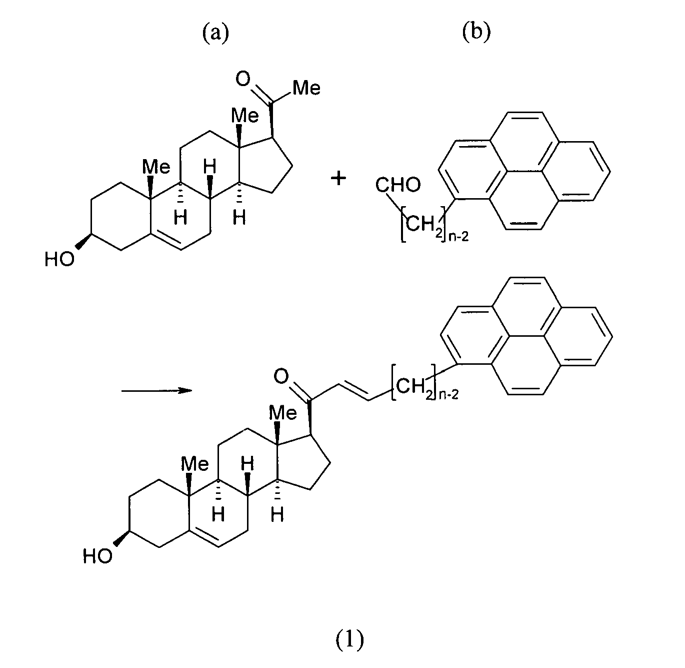
b) die Reaktion der im vorigen Schritt erhaltenen Verbindung der Formel (1) in Anwesenheit von 1,5 Äquivalenten Ac₂O, 10 Mol % DMAP, 1 Äquivalent Et₃N, in CH₂Cl₂ während 3 Stunden, was zum Erhalten der folgenden Verbindung der Formel (2) führt : 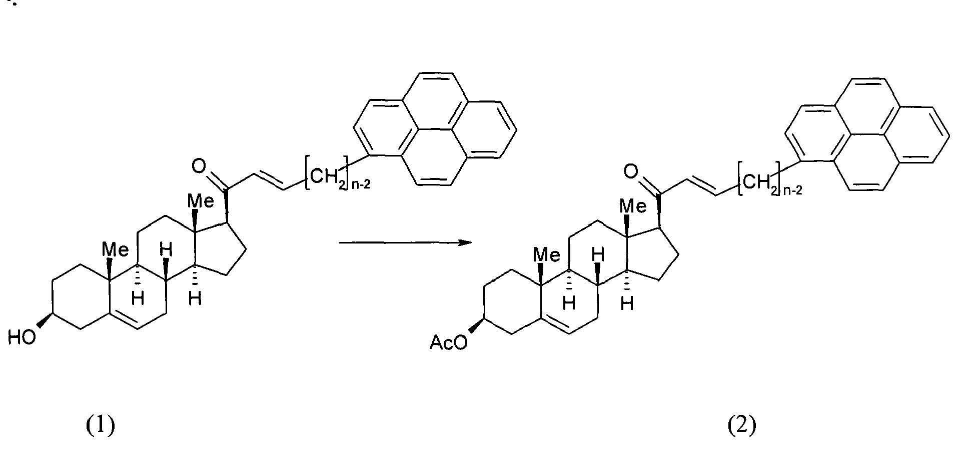
c) die Reaktion der im vorigen Schritt erhaltenen Verbindung der Formel (2) in Anwesenheit von 2 Äquivalenten TiCl₄, 1,5 Äquivalenten Et₃SiH, in CH₂Cl₂ während 45 Minuten bei Raumtemperatur, was zum Erhalten der folgenden Verbindung (3) führt : 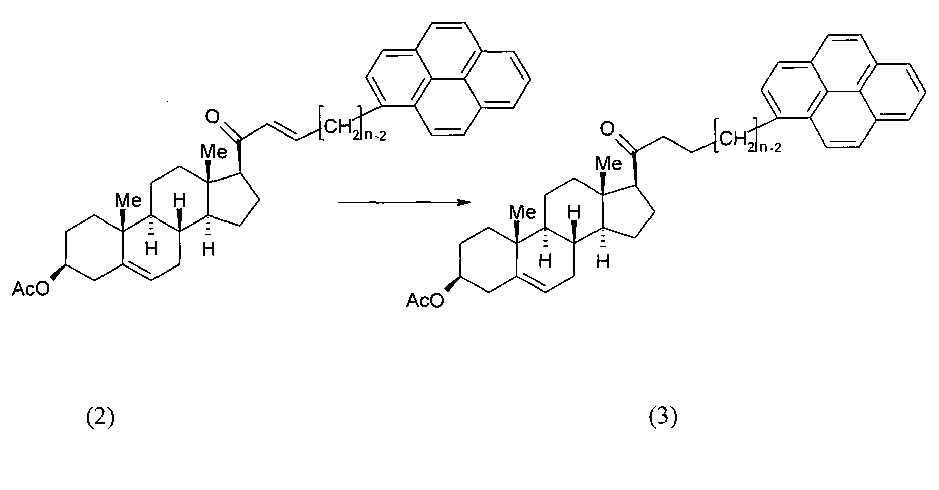
d) die Reaktion der im vorigen Schritt erhaltenen Verbindung der Formel (3) in Anwesenheit von MeONa, in CH₂Cl₂ während 4 Stunden bei Raumtemperatur, was zur folgenden Verbindung der Formel (4) führt : 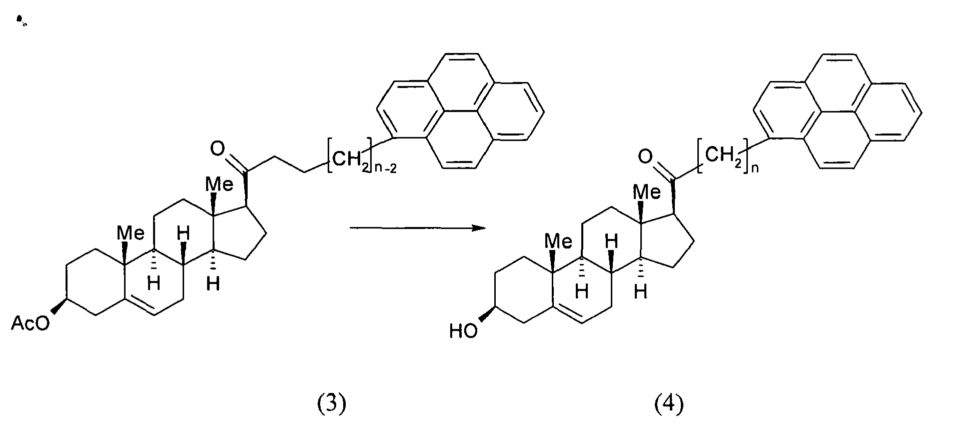
e) gegebenenfalls die Reaktion der im vorigen Schritt erhaltenen Verbindung der Formel (4) in Anwesenheit von TBDMSCI, Imidazol, in CH₂Cl₂ während 12 Stunden bei Raumtemperatur, was zum Erhalten der folgenden Verbindung der Formel (5) führt : 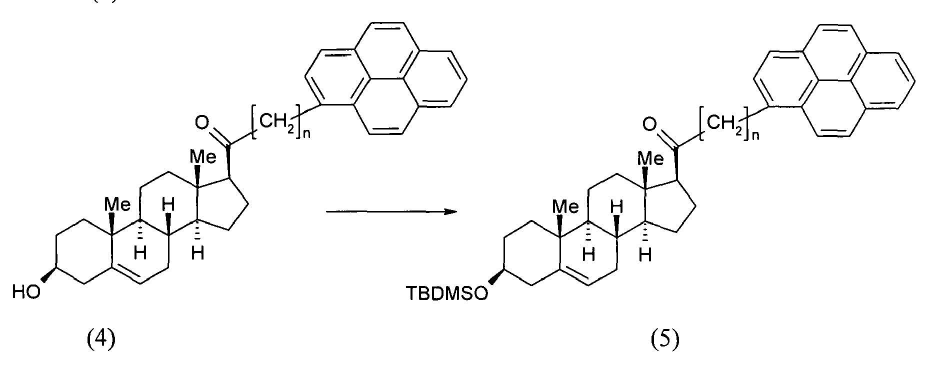
e) die Reaktion der im vorigen Schritt erhaltenen Verbindung der Formel (5) in Anwesenheit von 2 Äquivalenten CH₂=PPh₃, in einer Mischung von THF/DMSO, während 12 Stunden bei 75°C, was zum Erhalten der folgenden Verbindung der Formel (6) führt : 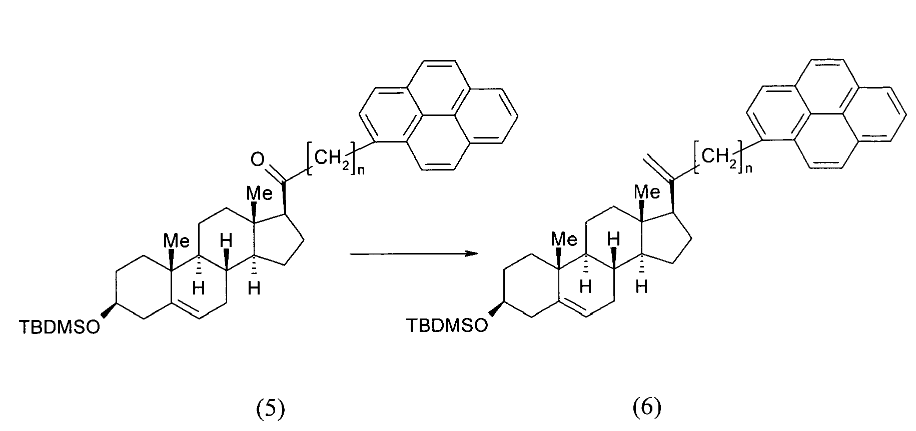
g) die Reaktion der im vorigen Schritt erhaltenen Verbindung der Formel (6) in Anwesenheit von 5 Äquivalenten TBAF, in THF, während 12 Stunden bei 65°C, was zur folgenden Verbindung der Formel (7) führt :
